# EUROPEAN PATENT SPECIFICATION

(11) **EP 3 201 178 B1**
(45) Date of publication and mention of the grant of the patent: **23.10.2019**
(21) Application number: 15782000.2
(22) Date of filing: 29.09.2015
(51) Int. Cl.: C07D 213/71, A61K 31/496, A61P 25/00

(54) **ARYLALKYLPIPERAZINES FOR USE AS NEUROPROTECTIVE AGENTS**
ARYLALKYLPIPERAZINE ZUR VERWENDUNG ALS NEUROPROTEKTIVUM
ARYLALKYLPIPÉRAZINES DESTINÉES À ÊTRE UTILISÉES EN TANT QU'AGENTS NEUROPROTECTEURS

(30) Priority: 29.09.2014 GB 201417168; 24.03.2015 GB 201504956
(43) Date of publication of application: 09.08.2017
(73) Proprietor: University of Hertfordshire Higher Education Corporation, Hatfield Hertfordshire AL10 9AB (GB)
(72) Inventor: IRAVANI, Mahmoud, London Greater London SW12 0JX (GB); SOSKIC, Vukic, London Greater London N3 1HP (GB)
(74) Representative: CSY Herts
(86) International application number: PCT/GB2015/052822
(87) International publication number: WO 2016/051155

(56) References cited:
- WO-A1-03/029233
- WO-A1-2004/041793
- WO-A1-2012/069428
- DATABASE Chemcats [Online] 15 September 2014 (2014-09-15), XP002751848, Database accession no. 1711816420
- DATABASE Chemcats [Online] 1 January 2015 (2015-01-01), XP002751849, Database accession no. 1080065424

## Description

### Field of the Invention

The present invention relates to novel arylpiperazines, in particular novel arylsulphonamide arylpiperazines, including the synthesis thereof and their applicability for use in the treatment or prophylaxis of a disease, in particular for use as neuroprotective agents, in treatment of neurological and neuropsychiatric disorders and in the treatment of diseases associated with, accompanied by or caused by mitochondrial stress.

### Background of the Invention

N-arylpiperazines are known in the art for having high affinity to numerous ligands suitable for different G protein coupled receptors (GPCRs) (Sukalovic et al., 2005).

Many N-arylpiperazines are biologically active and the N-arylpiperazine subunit can be found in pharmacologically compounds related to serotonin ligands, calcium blockers, antipsychotic drugs, antihypertensive drugs, and acetylcholinesterase inhibitory activity.

In particular N-arylpiperazines are known to have antipsychotic or antidepressant properties, and are capable of interacting with dopamine and serotonin receptor ligands or acetylcholinesterase inhibitors (Caccia, 2007).

Moreover, various compounds with N-arylpiperazine moiety have been found to exhibit direct neuroprotective effects in vitro (Druse et al., 2005; Koprivica et al., 2011; Park et al., 2011; Park et al., 2009). One such arylpiperazine derivative, aripiprazole, a second generation antipsychotic and partial agonist of D2 dopamine receptor, was found to increase the levels of neuroprotective brain-derived neurotrophic factor (BDNF) and antiapoptotic Bcl-2 protein whilst at the same time reducing the proapoptotic action of glycogen synthase kinase 3β in SH-SY5Y cells (Park et al., 2009). Aripiprazole has also been found to protect PC12 neuronal cell line from N-methyl-4-phenylpyridinium-induced oxidative stress (Park et al., 2011), as well as rat embryonic cortical neurons and rat dopaminergic neurons in mesencephalic cultures from glutamate-induced toxicity (Koprivica et al., 2011; Matsuo et al., 2010). Another arylpiperazine derivative, buspirone, which is a serotonin agonist, has been found to protect fetal rhombencephalic rat neurons from ethanol-induced apoptosis through activation of Akt (Druse et al., 2005).

In the USA, each year more than 60,000 new cases of Parkinson's are diagnosed. In the UK, it is estimated to affect 100-180 people per 100,000 head of the population. As the incidence of Parkinson's increases with aging, with 2-4% of those over the age 60 being affected, with the increase of ageing population the number of Parkinson's disease sufferers is likely to rise. The main treatments provide symptomatic relief only. The most commonly used first line of treatment for Parkinson's is the administration of L-dopa (levodopa). This may be administered in combination with a peripheral decarboxylase inhibitor such as carbidopa, which prolongs the effects of L-dopa by slowing the conversion of L-dopa to dopamine in the blood stream. However, long-term use of L-dopa causes abnormal hyperkinetic involuntary movements (dyskinesia) as a significant side effect. In fact, at least 40% of Parkinson's sufferers treated with L-dopa and a peripheral decarboxylase inhibitor develop dyskinesia within 5 years. Alternatively, dopamine agonists may be used which, whilst effective, may cause nausea, vomiting and psychiatric complications.

The causes of L-dopa induced dyskinesia are currently unknown. It is thought that a priming phenomenon occurs which causes the abnormal movements associated with dyskinesia. The therapeutic strategies for treatment therefore involve both preventing this priming process from occurring, as well as avoiding the expression of dyskinesia in patients where priming has occurred.

Muscarinic and glutamatergic antagonist, amantadine, is the only clinically approved treatment for dyskinesia reduction, but high doses are require, it is poorly tolerated and has been found to be ineffective in the long term. There is thus a need for an effective regime for inhibiting or treating dyskinesia, which can be carried out without affecting anti-Parkinson's treatments.

Possible treatments, which have been employed in the treatment of Parkinson's include opioid antagonists (e.g. naloxone) and agonists (e.g. morphine), alpha-2 adrenoceptor antagonists (idozoxan, fipamezole), 5-HT1a receptor agonists (sarizotan), 5-HT2a/c receptor antagonists (quetiapine), cannabinoid receptor agonists (e.g. nabilone), magnesium sulphate, 3,4-methylenedioxymethamphetamine (ecstasy), nicotine and the anti-convulsant, levatiracetam. However, none of the above treatments have been effective or useful in late stage clinical trials.

WO2004041793 discloses substituted piperazine derivatives for the treatment of CNS disorders.

### Disclosure of the Invention (Only subject matter covered bv the scope of the claims forms part of the invention).

In summary herein are presented a group of novel N-arylpiperazines with neuroprotective properties.

The inventors had identified N-{4-[2-(4-phenyl-piperazin-1-yl)ethyl]-phenyl}-picolinamide (1) and N-{3-[2-(4-phenyl-piperazin-1-yl)ethyl]-phenyl}-picolinamide (2) in their previous research of synthesized dopaminergic ligands with an arylpiperazine moiety as the most active in preventing mitochondrial damage and apoptosis induced by nitric oxide and 6-hydroxy dopamine in SH-SY5Y cells (Tovilovic et al., 2012, Tovilovic et al., 2013).

It has been discovered since, by the inventors, that those arylpiperazines modulate apoptotic and autophagic responses induced by neurotoxin 6-OHDA in human SH-SY5Y neuron-like cells. In rat model of EAE the described compounds demonstrated BBB penetrability and in vivo activity against induced neuropathy.

Starting from the structural scaffolding of arylpiperazines N-{4-[2-(4-phenyl-piperazin-1-yl)ethyl]-phenyl}-picolinamide (1) and N-{3-[2-(4-phenyl-piperazin-1-yl)ethyl]-phenyl}-picolinamide (2), bioisosteric heteroaryl sulphonamides were prepared and their cytoprotective activity was determined in a primary culture of neuronal cells.

The sulphonamide moiety was chosen because it is not associated with any toxicity and is known to be present in a wide range of compounds for the treatment of various diseases.

Among tested sulphonamides N-{4-[2-(4-phenyl-piperazin-1-yl)ethyl]-phenyl}-picolinsulphonamide and N-{3-[2-(4-phenyl-piperazin-1-yl)ethyl]-phenyl}-nicotinsulphonamide appeared as most interesting candidates for further research.

A first aspect of the invention relates to a compound of the general formula A 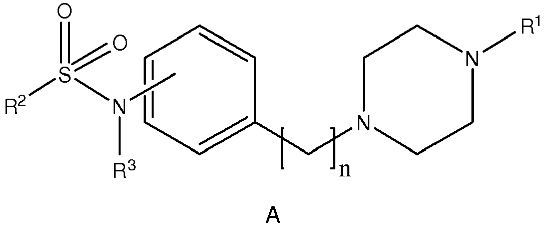 or a or a pharmaceutically acceptable salt or solvate thereof,
wherein:
R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³;
R² is selected from the group consisting of: C₆-C₁₀-aryl and C₅-C₇-heteroaryl; each optionally substituted with halogen, hydroxyl, OCOR³ amino, C₁-C₆-alkylamino, C₁-C₆-dialkyl- amino, C₁-C₆-(halo)alkyl, C₁-C₆-(halo)alkoxy and/or COOR³;
R³ is independently at each occurrence selected from the group consisting of: hydrogen and C₁-C₂-alkyl; and
n is an integer from 2 to 10 inclusive.

Preferably, a given moiety is substituted with one, two, three, four, or five independently selected substituents.

Preferably R¹ is phenyl optionally substituted with halogen, hydroxyl, C₁-C₆-(halo)alkyl, and/or C₁-C-(halo)alkoxy.

More preferably R¹ is phenyl, optionally substituted with halogen, hydroxyl, and/or C₁-C₆-alkoxy.

Preferably R² is selected from the group consisting of: phenyl and C₁-C₆-heteroaryl, each optionally substituted with halogen, hydroxyl, amino, C₁-C₆-(halo)alkyl, and/or C₁-C₆-(halo)alkoxy.

More preferably R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy.

Still more preferably R² is selected from the group consisting of: phenyl, 2-pyridyl, 3-pyridyl, and pyridazinyl, each optionally substituted with halogen, hydroxyl, amino and/or C₁-C₆-alkoxy.

Yet more preferably R² is selected from the group consisting of: phenyl, 2-pyridyl, and 3-pyridyl. The phenyl group may optionally be substituted with halogen, amino, and/or C₁-C₆-alkoxy. The 2-pyridyl or 3- pyridyl group may optionally be substituted with halogen, hydroxyl, amino, and/or C₁-C₆-alkoxy, preferably being monosubstituted in the ortho-position.

Preferably R³ is hydrogen.

Preferably n is an integer from 2 to 6 inclusive. More preferably n is 2.

Preferably R¹ is phenyl, which is optionally substituted with halogen, hydroxyl, and/or C₁-C₆-alkoxy; R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy; and n is 2.

Still more preferably, R¹ is an unsubstituted phenyl; R² is selected from the group consisting of: phenyl, 2-pyridyl, 3-pyridyl, 4-pyridyl, pyridazinyl, pyrazinyl, each optionally substituted with hydroxyl; R³ is hydrogen; and n is 2.

In one alternative the invention relates to a compound of the general formula I. 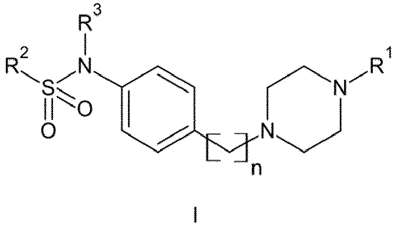 or a or a pharmaceutically acceptable salt or solvate thereof,
wherein:
R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³;
R² is selected from the group consisting of: C₆-C₁₀-aryl and C₅-C₇-heteroaryl; each optionally substituted with halogen, hydroxyl, OCOR³ amino, C₁-C₆-alkylamino, C₁-C₆-dialkyl- amino, C₁-C₆-(halo)alkyl, C₁-C₆-(halo)alkoxy and/or COOR³;
R³ is independently at each occurrence selected from the group consisting of: hydrogen and C₁-C₂-alkyl; and
n is an integer from 2 to 10 inclusive.

Preferably, a given moiety is substituted with one, two, three, four, or five independently selected substituents.

Preferably R¹ is phenyl optionally substituted with halogen, hydroxyl, C₁-C₆-(halo)alkyl, and/or C₁-C₆-(halo)alkoxy.

More preferably R¹ is phenyl, optionally substituted with halogen, hydroxyl, and/or C₁-C₆-alkoxy.

Preferably R² is selected from the group consisting of: phenyl and C₁-C₆-heteroaryl, each optionally substituted with halogen, hydroxyl, amino, C₁-C₆-(halo)alkyl, and/or C₁-C₆-(halo)alkoxy.

More preferably R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy.

Still more preferably R² is selected from the group consisting of: phenyl, 2-pyridyl, 3-pyridyl, and pyridazinyl, each optionally substituted with halogen, hydroxyl, amino and/or C₁-C₆-alkoxy.

Yet more preferably R² is selected from the group consisting of: phenyl, 2-pyridyl, and 3-pyridyl. The phenyl group may optionally be substituted with halogen, amino, and/or C₁-C₆-alkoxy. The 2-pyridyl or 3- pyridyl group may optionally be substituted with halogen, hydroxyl, amino, and/or C₁-C₆-alkoxy, preferably being monosubstituted in the ortho-position.

Preferably R³ is hydrogen.

Preferably n is an integer from 2 to 6 inclusive. More preferably n is 2.

Preferably R¹ is phenyl, which is optionally substituted with halogen, hydroxyl, and/or C₁-C₆-alkoxy; R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy; and n is 2.

Still more preferably, R¹ is an unsubstituted phenyl; R² is selected from the group consisting of: phenyl, 2-pyridyl, 3-pyridyl, 4-pyridyl, pyridazinyl, pyrazinyl, each optionally substituted with hydroxyl; R³ is hydrogen; and n is 2.

Still more preferably R¹ is unsubstituted phenyl; R² is selected from the group consisting of: phenyl, 2-pyridyl, 3-pyridyl, pyridazinyl, each unsubstituted or substituted with hydroxyl; R³ is hydrogen; and n is 2.

In one alternative the invention relates to a compound of the general formula II. 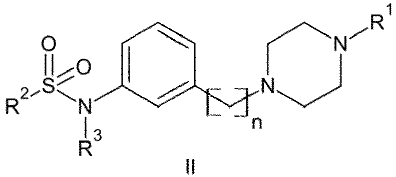 or a or a pharmaceutically acceptable salt or solvate thereof,
wherein:
R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³;
R² is selected from the group consisting of: C₆-C₁₀-aryl and C₅-C₇-heteroaryl; each optionally substituted with halogen, hydroxyl, OCOR³ amino, C₁-C₆-alkylamino, C₁-C₆-dialkyl- amino, C₁-C₆-(halo)alkyl, C₁-C₆-(halo)alkoxy and/or COOR³;
R³ is independently at each occurrence selected from the group consisting of: hydrogen and C₁-C₂-alkyl;
and n is an integer from 2 to 10 inclusive.

Preferably, a given moiety is substituted with one, two, three, four, or five independently selected substituents.

Preferably R¹ is phenyl optionally substituted with halogen, hydroxyl, C₁-C₆-(halo)alkyl, and/or C₁-C₆-(halo)alkoxy.

More preferably R¹ is phenyl, optionally substituted with halogen, hydroxyl, and/or C₁-C₆-alkoxy.

Preferably R² is selected from the group consisting of: phenyl and C₁-C₆-heteroaryl, each optionally substituted with halogen, hydroxyl, amino, C₁-C₆-(halo)alkyl, and/or C₁-C₆-(halo)alkoxy.

More preferably R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy.

Still more preferably R² is selected from the group consisting of: phenyl, 2-pyridyl, 3-pyridyl, and pyridazinyl, each optionally substituted with halogen, hydroxyl, amino and/or C₁-C₆-alkoxy.

Yet more preferably R² is selected from the group consisting of: phenyl, 2-pyridyl, and 3-pyridyl. The phenyl group may optionally be substituted with halogen, amino, and/or C₁-C₆-alkoxy. The 2-pyridyl or 3- pyridyl group may optionally be substituted with halogen, hydroxyl, amino, and/or C₁-C₆-alkoxy, preferably being monosubstituted in the ortho-position.

Preferably R³ is hydrogen.

Preferably n is an integer from 2 to 6 inclusive. More preferably n is 2.

Preferably R¹ is phenyl, which is optionally substituted with halogen, hydroxyl, and/or C₁-C₆-alkoxy; R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy; and n is 2.

Still more preferably, R¹ is an unsubstituted phenyl; R² is selected from the group consisting of: phenyl, 2-pyridyl, 3-pyridyl, 4-pyridyl, pyridazinyl, pyrazinyl, each optionally substituted with hydroxyl; R³ is hydrogen; and n is 2.

Still more preferably R¹ is an unsubstituted phenyl; R² is selected from the group consisting of: phenyl, 2-pyridyl, and 3-pyridyl, each optionally substituted with hydroxyl, with phenyl preferably being unsubstituted and 2-pyridyl and 3- pyridyl preferably being unsubstituted or monosubstituted in the ortho- position; R³ is hydrogen; and n is 2.

In one alternative the invention relates to a compound of the general formula III. 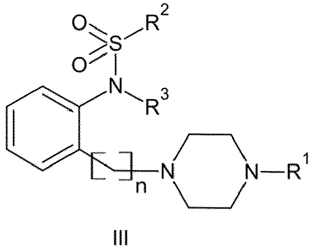 or a or a pharmaceutically acceptable salt or solvate thereof,
wherein:
R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³;
R² is selected from the group consisting of: C₆-C₁₀-aryl and C₅-C₇-heteroaryl; each optionally substituted with halogen, hydroxyl, OCOR³ amino, C₁-C₆-alkylamino, C₁-C₆-dialkyl- amino, C₁-C₆-(halo)alkyl, C₁-C₆-(halo)alkoxy and/or COOR³;
R³ is independently at each occurrence selected from the group consisting of: hydrogen and C₁-C₂-alkyl; and
n is an integer from 2 to 10 inclusive.

Preferably, a given moiety is substituted with one, two, three, four, or five independently selected substituents.

Preferably R¹ is selected from the group consisting of: C₁-C₆-alkyl, phenyl, and nitrogen-containing C₆-heteroaryl, each optionally substituted with halogen, hydroxyl, C₁-C₆-(halo)alkyl, and/or C₁-C₆-(halo)alkoxy.

More preferably R¹ is phenyl, optionally substituted with halogen, hydroxyl, and/or C₁-C₆-alkoxy.

Preferably R² is phenyl optionally substituted with halogen, hydroxyl, amino, C₁-C₆-(halo)alkyl, and/or C₁-C₆-(halo)alkoxy.

More preferably R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy.

Still more preferably R² is selected from the group consisting of: phenyl, 2-pyridyl, 3-pyridyl, and pyridazinyl, each optionally substituted with halogen, hydroxyl, amino and/or C₁-C₆-alkoxy.

Yet more preferably R² is selected from the group consisting of: phenyl, 2-pyridyl, and 3-pyridyl. The phenyl group may optionally be substituted with halogen, amino, and/or C₁-C₆-alkoxy. The 2-pyridyl or 3- pyridyl group may optionally be substituted with halogen, hydroxyl, amino, and/or C₁-C₆-alkoxy, preferably being monosubstituted in the ortho-position.

Preferably R³ is hydrogen.

Preferably n is an integer from 2 to 6 inclusive. More preferably n is 2.

Preferably R¹ is phenyl, which is optionally substituted with halogen, hydroxyl, and/or C₁-C₆-alkoxy; R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy; and n is 2.

Still more preferably, R¹ is an unsubstituted phenyl; R² is selected from the group consisting of: phenyl, 2-pyridyl, 3-pyridyl, 4-pyridyl, pyridazinyl, pyrazinyl, each optionally substituted with hydroxyl; R³ is hydrogen; and n is 2.

More preferably, the compound according to the invention is selected from the group consisting of:
N-{4-[2-(4-phenyl-piperazin-1-yl)-ethyl]-phenyl}-benzsulphonamide,
N-{3-[2-(4-phenyl-piperazin-1-yl)-ethyl]-phenyl}-benzsulphonamide,
N-{2-[2-(4-phenyl-piperazin-1-yl)-ethyl]-phenyl}-benzsulphonamide,
N-{4-[2-(4-phenyl-piperazin-1-yl)-ethyl]-phenyl}-2-pyridylsulphonamide,
N-{3-[2-(4-phenyl-piperazin-1-yl)-ethyl]-phenyl}-2-pyridylsulphonamide,
N-{2-[2-(4-phenyl-piperazin-1-yl)-ethyl]-phenyl}-2-pyridylsulphonamide,
N-{4-[2-(4-phenyl-piperazin-1-yl)-ethyl]-phenyl}-3-pyridylsulphonamide,
N-{3-[2-(4-phenyl-piperazin-1-yl)-ethyl]-phenyl}-3-pyridylsulphonamide,
N-{2-[2-(4-phenyl-piperazin-1-yl)-ethyl]-phenyl}-3-pyridylsulphonamide,
and pharmaceutically acceptable salts or solvates thereof.

Most preferably, the compound of the general formula A, I, II or III is selected from the group consisting of: N-{2-[2-(4-phenyl-piperazin-1-yl)-ethyl]-phenyl}-2-pyridyl-sulphonamide (IIIa), N-{2-[2-(4-phenyl-piperazin-1-yl)-ethyl]-phenyl}-3-pyridyl-sulphonamide (iIIb) N-{3-[2-(4-phenyl-piperazin-1-yl)-ethyl]-phenyl}-2-pyridyl-sulphonamide (IIa), N-{3-[2-(4-phenyl-piperazin-1-yl)-ethyl]-phenyl}-3-pyridyl-sulphonamide (IIb) N-{4-[2-(4-phenyl-piperazin-1-yl)-ethyl]-phenyl}-2-pyridyl-sulphonamide (Ia) and N-{4-[2-(4-phenyl-piperazin-1-yl)-ethyl]-phenyl}-3-pyridyl-sulphonamide (Ib) or a pharmaceutically acceptable salt or solvate thereof.

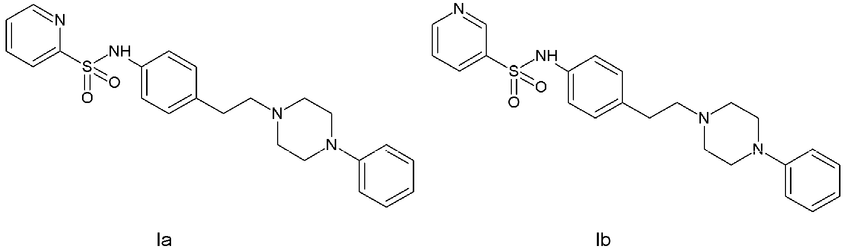

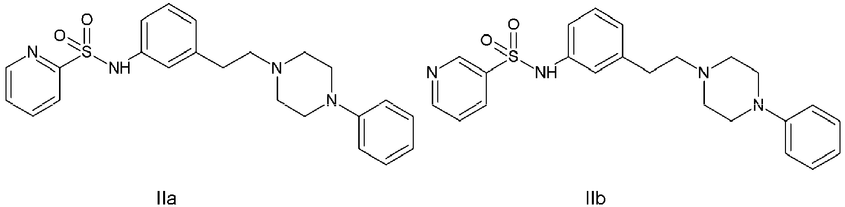

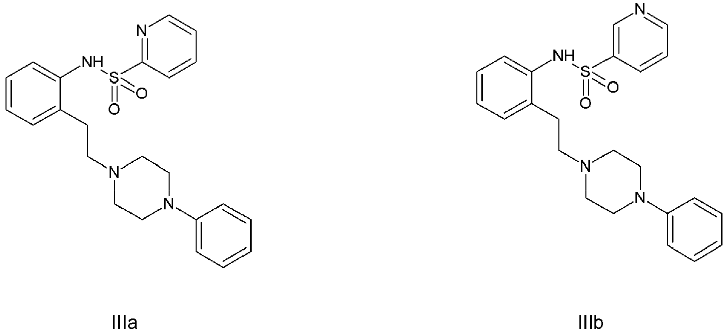

Preferably, the compounds of the general formula A, I, II or III have neuroprotective activity. In particular, the compounds of the general formula A, I, II or III are preferably capable of protecting human neuroblastoma cells from oxidative stress induced by 6-hydroxy dopamine.

According to a second aspect of the present invention there is provided a process for the manufacture of a compound of the general formula A, I, II or III, which comprises the steps of:
(i) reacting an arylcarboxylic acid of general formula IV a), IV b), or IV c) 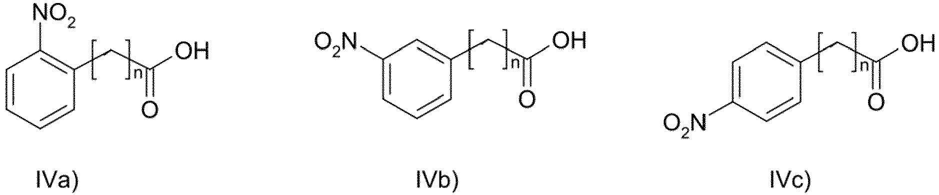 with an amine of general formula V 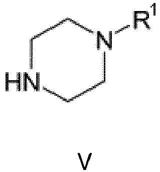 to give a compound of general formula VIa), VIb), or VIc); 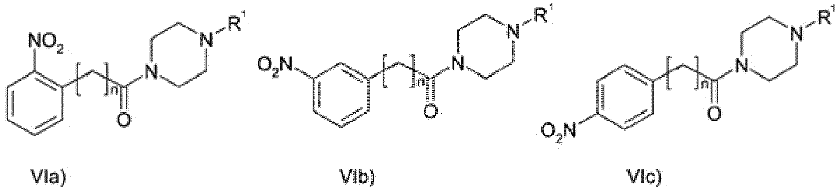
(ii) reducing said compound of formula VI a), VI b) or VI c) with a suitable reducing agent;
(iii) hydrogenating the product of step (ii);
(iv) reacting the product of step (iii) with a arylsulphonic acid chloride of the general formula R²SO₂Cl; and
(v) optionally isolating the resulting compound of formula A, I, II, or III,
wherein:
R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³;
R² is selected from the group consisting of: C₆-C₁₀-aryl and C₅-C₇-heteroaryl; each optionally substituted with halogen, hydroxyl, OCOR³ amino, C₁-C₆-alkylamino, C₁-C₆-dialkyl- amino, C₁-C₆-(halo)alkyl, C₁-C₆-(halo)alkoxy and/or COOR³;
R³ is independently at each occurrence selected from the group consisting of: hydrogen and C₁-C₂-alkyl; and
n is an integer from 1 to 9 inclusive.

Preferably the arylcarboxylic acid of step (i) has the general formula VII 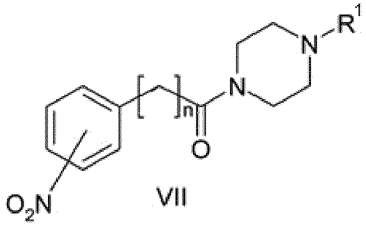 wherein R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³; and n is an integer from 1 to 9 inclusive.

Preferably the amine of step (i) has the general formula VIII 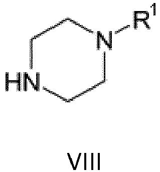 wherein R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³.

Preferably the reaction product of step (i) has the general formula IX 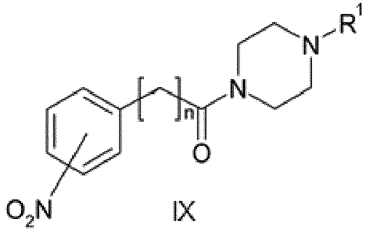 wherein R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³.

More preferably R¹ is phenyl, R² is selected from phenyl, 2-pyridyl, 3-pyridyl, 4-pyridyl, pyridazinyl, and pyrazinyl, each optionally substituted with hydroxyl; R³ is hydrogen at each occurrence; and n is 1.

Suitable reducing agents for use in the reduction step (ii) include but are not limited to diborane (B₂H₆), lithium aluminium hydride (LAH; LiAlH₄), and diisobutylaluminium hydride (DIBAL).

Suitable catalysts for hydrogenating the nitro group (NO₂) in step (iii) include but are not limited to transition metals of groups 8, 9, 10 and 11 of the periodic table, in particular nickel (e.g. Raney nickel), platinum, palladium, rhodium, and ruthenium.

According to a third aspect of the present invention there is provided a pharmaceutical composition comprising one or more compounds of the general formula A, I, II or III as described above and pharmaceutically acceptable excipients, adjuvants, diluents and/or carriers.

Suitable excipients include but are not limited to; disintegrators, binders, fillers, and lubricants.

Suitable disintegrators include but are not limited to; agar-agar, algins, calcium carbonate, cellulose, colloid silicon dioxide, gums, magnesium aluminium silicate, methylcellulose, and starch.

Suitable binders include but are not limited to hydroxymethyl cellulose, hydroxypropylcellulose, microcrystalline cellulose, and polyvinylpyrrolidone.

Suitable fillers include but are not limited to calcium carbonate, calcium phosphate, tribasic calcium sulphate, calcium carboxymethylcellulose, cellulose, dextrin, dextrose, fructose, lactitol, lactose, magnesium carbonate, magnesium oxide, maltitol, maltodextrins, maltose, sorbitol, starch, sucrose, sugar, and xylitol.

Suitable lubricants include but are not limited to agar, ethyl laureate, ethyl oleate, glycerin, glyceryl palmitostearate, glycols, hydrogenated vegetable oil, magnesium oxide, mannitol, poloxamer, sodium benzoate, sodium lauryl sulphate, sodium stearyl, sorbitol, stearates, and talc.

Suitable adjuvants include but are not limited to buffer substances, colorants, consistency-improving agents, diluents, emollients, flavour-improving agents, preservatives, salts for varying the osmotic pressure, solubilisers, stabilisers, wetting and emulsifying agents, masking agents and antioxidants.

Suitable carriers include but are not limited to magnesium carbonate, magnesium stearate, talc, sugar, lactose, pectin, dextrin, starch, gelatine, tragacanth, methylcellulose, sodium carboxymethylcellulose, a low melting-point wax, cocoa butter, water, alcohols, polyols, glycerol, vegetable oils and the like.

The pharmaceutical composition may also comprise at least one further active agent, which may include one or more further organic or inorganic molecule.

The composition may be used alone, without further medication. Alternatively, the composition may be used in combination with other medicaments, such as medicaments for treating neurodegenerative diseases such as Alzheimer's disease, Parkinson's disease, ALS, stroke, and age-related macular degeneration. Mitochondria are cellular organelles composed of two membranes. An inner mitochondrial membrane (IMM), which is organized into so-called cristae, is surrounded by an outer membrane, which encloses the entire organelle. The space surrounded by the IMM is called matrix and harbours the majority of the mitochondrial proteins, as well as the mitochondrial genome. Between the two membranes, there is an inter-membrane space.

The most prominent function of mitochondria is their role in the regulation of cellular metabolism and, most importantly, in the oxidative phosphorylation, a central step in the production of energy in the form of ATP. The content of mitochondria differs according to the cell type. Cells which have high energy consumption, such as muscle and nerve cells, are particularly rich in mitochondria.

A problem in the process of oxidative phosphorylation is that a small part of the electrons transported in the respiratory chain is responsible for the formation of reactive oxygen species (ROS) such as the dioxide anion (O₂⁻.), also known as superoxide and hydrogen peroxide (H₂O₂). ROS lead to propagation of free radicals and can oxidize cellular lipids, nucleotide bases, and proteins. For example, O₂⁻ can react with nitric oxide (NO) to form toxic compounds such as peroxy nitrite (ONOO⁻). Inhibition of components of the mitochondrial respiratory chain may ultimately lead to apoptosis of cells.

A state where cells, e.g. human cells, produce increased amounts of oxidants, e.g. ROS, leading to an increased release of free radicals and resulting in cellular degeneration is referred to as oxidative or mitochondrial stress. Mitochondrial stress seems to be involved in several disorders, including neurodegenerative and cardiovascular diseases.

According to a fourth aspect of the present invention there is provided a compound of the general formula A, I, II or III for use as a medicament. In one alternative for use as a human medicament, in another alternative for use as a veterinary medicament.

In one alternative the medicament is for use as a neuroprotective agent.
In another alternative the medicament is for use in the treatment or prophylaxis of neurological and neuropsychiatric disorders.

In a further alternative the medicament is for use in the treatment or prophylaxis of diseases, disorders or conditions associated with, accompanied by or caused by mitochondrial stress.

In yet another alternative the medicament is for use in the treatment of a neurodegenerative disorder. Preferably, said neurodegenerative disorder is selected from amyotrophic lateral sclerosis (ALS, also known as Lou Gehrig's disease), Alzheimer's disease, Parkinson's disease, stroke, and age-related macular degeneration.

In another alternative the medicament is for use in the treatment of pathological conditions related to mitochondrial dysfunction and dysfunction of serotonin and dopamine transmitter systems. The medicament acts on the dopamine and serotonin receptors and the antiapoptotic property of the medicament is mediated through mitochondrial dysfunction.

According to a fifth aspect of the present invention there is provided a compound of the general formula A, I, II or III for use in the treatment or prophylaxis of a disease.

In one alternative the compound is for use as a neuroprotective agents.

In another alternative the compound is for use in the treatment or prophylaxis of neurological and neuropsychiatric disorders.

In a further alternative the compound is for use in the treatment or prophylaxis of diseases, disorders or conditions associated with, accompanied by or caused by mitochondrial stress.

In yet another alternative the compound is for use in the treatment of a neurodegenerative disorder. Preferably, said neurodegenerative disorder is selected from amyotrophic lateral sclerosis (ALS, also known as Lou Gehrig's disease), Alzheimer's disease, Parkinson's disease, stroke, and age-related macular degeneration.
In another alternative the compound is for use in the treatment of pathological conditions related to mitochondrial dysfunction and dysfunction of serotonin and dopamine transmitter systems. The compound acts on the dopamine and serotonin receptors and the antiapoptotic property of the medicament is mediated through mitochondrial dysfunction.

According to a sixth aspect of the present invention there is provided a compound of the general formula A, I, II or III for use in a method of treatment or prophylaxis of a disease.

In one alternative the compound is for use in a method as a neuroprotective agent.

In another alternative the compound is for use in a method of treatment or prophylaxis of neurological and neuropsychiatric disorders.

In a further alternative the compound is for use in a method of treatment or prophylaxis of diseases, disorders or conditions associated with, accompanied by or caused by mitochondrial stress.

In yet another alternative the compound is for use in a method of treatment of a neurodegenerative disorder. Preferably, said neurodegenerative disorder is selected from amyotrophic lateral sclerosis (ALS, also known as Lou Gehrig's disease), Alzheimer's disease, Parkinson's disease, stroke, and age-related macular degeneration.

In another alternative the compound is for use in a method of treatment of pathological conditions related to mitochondrial dysfunction and dysfunction of serotonin and dopamine transmitter systems. The compound acts on the dopamine and serotonin receptors and the antiapoptotic property of the medicament is mediated through mitochondrial dysfunction.

According to a seventh aspect of the present invention there is provided a method for manufacturing a medicament intended for the treatment or prophylaxis of a disease, characterised in that a compound of the general formula A, I, II or III is used.

In one alternative the medicament intended for use as a neuroprotective agent.
In another alternative the medicament intended for use in the treatment or prophylaxis of neurological and neuropsychiatric disorders.

In a further alternative the medicament intended for use in the treatment or prophylaxis of diseases, disorders or conditions associated with, accompanied by or caused by mitochondrial stress.

In yet another alternative the medicament is for use in the treatment of a neurodegenerative disorder. Preferably, said neurodegenerative disorder is selected from amyotrophic lateral sclerosis (ALS, also known as Lou Gehrig's disease), Alzheimer's disease, Parkinson's disease, stroke, and age-related macular degeneration.

In another alternative the medicament is for use in the treatment of pathological conditions related to mitochondrial dysfunction and dysfunction of serotonin and dopamine transmitter systems. The medicament acts on the dopamine and serotonin receptors and the antiapoptotic property of the medicament is mediated through mitochondrial dysfunction.

According to an eighth aspect of the present invention there is provided a pharmaceutical composition comprising a compound of the general formula A, I, II or III as described above and pharmaceutically acceptable excipients, adjuvants, diluents and/or carriers for use in the treatment or prophylaxis of a disease.

In one alternative the pharmaceutical composition is for use as a neuroprotective agent.

In another alternative the pharmaceutical composition is for use in the treatment or prophylaxis of neurological and neuropsychiatric disorders.

In a further alternative the pharmaceutical composition is for use in the treatment or prophylaxis of diseases, disorders or conditions associated with, accompanied by or caused by mitochondrial stress.

In yet another alternative the pharmaceutical composition is for use in the treatment of a neurodegenerative disorder. Preferably, said neurodegenerative disorder is selected from amyotrophic lateral sclerosis (ALS, also known as Lou Gehrig's disease), Alzheimer's disease, Parkinson's disease, stroke, and age-related macular degeneration.

In another alternative the pharmaceutical composition is for use in the treatment of pathological conditions related to mitochondrial dysfunction and dysfunction of serotonin and dopamine transmitter systems. The pharmaceutical composition acts on the dopamine and serotonin receptors and the antiapoptotic property of the medicament is mediated through mitochondrial dysfunction.

According to an ninth aspect of the present invention there is provided a pharmaceutical composition comprising a compound of the general formula A, I, II or III as described above and pharmaceutically acceptable excipients, adjuvants, diluents and/or carriers for use in a method of treatment or prophylaxis of a disease.

In one alternative the pharmaceutical composition is for use as a neuroprotective agents.

In another alternative the pharmaceutical composition is for use in a method of treatment or prophylaxis of neurological and neuropsychiatric disorders.

In a further alternative the pharmaceutical composition is for use in a method of treatment or prophylaxis of diseases, disorders or conditions associated with, accompanied by or caused by mitochondrial stress.

In yet another alternative the pharmaceutical composition is for use in a method of treatment of a neurodegenerative disorder. Preferably, said neurodegenerative disorder is selected from amyotrophic lateral sclerosis (ALS, also known as Lou Gehrig's disease), Alzheimer's disease, Parkinson's disease, stroke, and age-related macular degeneration.

In another alternative the pharmaceutical composition is for use in a method of treatment of pathological conditions related to mitochondrial dysfunction and dysfunction of serotonin and dopamine transmitter systems. The pharmaceutical composition acts on the dopamine and serotonin receptors and the antiapoptotic property of the medicament is mediated through mitochondrial dysfunction. According to a tenth aspect of the present invention there is provided compounds for use in a method of treatment of a disease or condition of the human or animal body comprising administering a therapeutically effective amount of a compound of the general formula A, I, II or III or a pharmaceutical composition as described above.

In one alternative the method of treatment or prophylaxis is of neurological and neuropsychiatric disorders.

In a further alternative the method of treatment or prophylaxis is of diseases, disorders or conditions associated with, accompanied by or caused by mitochondrial stress.

In yet another alternative the method of treatment or prophylaxis is of treatment of a neurodegenerative disorder. Preferably, said neurodegenerative disorder is selected from amyotrophic lateral sclerosis (ALS, also known as Lou Gehrig's disease), Alzheimer's disease, Parkinson's disease, stroke, and age-related macular degeneration.

In particular, the compound of general formula A, I, II, or III, or a pharmaceutical composition comprising a compound of general formula A, I, II, or III, is for use in the treatment of a disease, disorder or condition associated with, accompanied by or caused by mitochondrial stress.

The present invention also relates to a method for the treatment of a disease, disorder or condition associated with, accompanied by or caused by mitochondrial stress, comprising administering a pharmaceutically effective amount of a of formula A, I, II or III to a subject in need thereof.

### Definitions

A pharmaceutically acceptable salt relates to salts or complexes of a compound of formula A, I, II, or III. Examples of such salts include, but are not limited to, base addition salts formed by reaction of a compound of formula A, I, II, or III, with an organic or inorganic base, e.g. ammonia or a hydroxide, carbonate or bicarbonate of a metal cation, which is preferably selected from alkali metals (e.g. sodium, potassium or lithium), and alkaline earth metals (e.g. calcium or magnesium), or with an organic primary, secondary or tertiary alkyl amine. Also encompassed are acid addition salts formed with inorganic acids (e.g. hydrochloric acid, hydrobromic acid, nitric acid, sulphuric acid, phosphoric acid), as well as salts formed with organic acids, e.g. aliphatic monocarboxylic and dicarboxylic acids, aromatic acids, and sulfonic acids. Non-limiting examples of such acids include acetic acid, benzoic acid, (+)-camphor-10- sulfonic acid, citric acid, gluconic acid, lactic acid, methanesulfonic acid, propionic acid, oxalic acid, succinic acid, tartric acid, trifluoroacetic acid, and triphenylacetic acid.

A pharmaceutically acceptable solvate may be for example a hydrate.

The term (halo)alkyl as used herein relates to an alkyl group which optionally contains at least one halogen, e.g. F, Cl, Br or I substituent up to perhalogenation.

The term alkyl as used herein relates to a monovalent linear or branched, saturated or unsaturated hydrocarbon moiety, consisting of carbon and hydrogen atoms, wherein the number of carbon atoms is defined by a subscript number, e.g. C₁-C₁₂. Non-limiting examples of alkyl moieties include methyl, ethyl, ethenyl, ethynyl, ethinyl, propyl, isopropyl, allyl, n-butyl, isobutyl, tert-butyl, butenyl, hexyl, octyl, and dodecyl.

The term cycloalkyl means a monovalent saturated or partially unsaturated carbocyclic moiety, preferably consisting of one or two rings. Non-limiting examples include cyclopropyl, cyclopentyl, cyclopentenyl, cyclohexyl, cyclohexadienyl, decalinyl, norbornyl.

The term aryl as used herein relates to an unsaturated aromatic carbocyclic group of from 6 to 10 carbon atoms having a single ring or two condensed rings. Non-limiting examples of aryl moieties include phenyl and naphthyl.

The term heteroaryl as used herein relates to a monocyclic radical of 5 to 7 ring atoms containing one, two, or three ring heteroatoms selected from nitrogen, oxygen, and sulphur, the remaining ring atoms being carbon. Non-limiting examples of heteroaryl moieties include furanyl, imidazolyl, isoxazolyl, oxazolyl, pyridyl, pyridazinyl, pyrazinyl, thiazolyl, and thiophenyl.

The term alkoxy as used herein relates to a moiety of the formula -OR, wherein R is an alkyl moiety as defined herein. Non-limiting examples of alkoxy moieties include methoxy, ethoxy, and isopropoxy.

The term substituted as used herein means that one or more functional groups, substituents, are attached to one or more carbon atoms of an alkyl, cycloalkyl, aryl, or heteroaryl moiety as defined herein.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

All compatible combinations of the embodiments described above are explicitly disclosed herein as if each combination was specifically and individually recited. Any sub-titles herein are included for convenience only, and are not to be construed as limiting the disclosure in any way.

The invention will now be further described with reference to the following non-limiting Figures and Examples. Other embodiments of the invention will occur to those skilled in the art in the light of these.

The disclosure of all references cited herein, inasmuch as it may be used by those skilled in the art to carry out the invention, is hereby specifically incorporated herein by cross- reference.

### Description of the Figures

Figure 1 illustrates Scheme 1, the synthetic route and chemical structures N-{4-[2-(4-aryl-piperazin-1-yl)-ethyl]-phenyl}-arylsulphonamides, N-{3-[2-(4-aryl-piperazin-1-yl)-ethyl]-phenyl}-arylsulphonamides and N-{2-[2-(4-aryl-piperazin-1-yl)-ethyl]-phenyl}-arylsulphonamides;
Figure 2 illustrates Scheme 2 the synthetic route and chemical structures of the N-{4-[2-(4-aryl-piperazin-1-yl)-methyl]-phenyl}-arylsulphonamides, N-{3-[2-(4-aryl-piperazin-1-yl)-methyl]-phenyl}-arylsulphonamides and N-{2-[2-(4-aryl-piperazin-1-yl)-methyl]-phenyl}-arylsulphonamides;
Figure 3 illustrates Table 1 illustrating the chemical structure and binding affinity of the some new arylpiperazine-sulphonamides for the D₂ DA and 5-HT_{1A} receptors;
Figure 4 illustrates the effect of compounds 1 and 2 on motor clinical scores;
Figure 5 illustrates the viability of rat ventral mesencephalic cells in culture following administration of proteasomal inhibitor lactacystin (5µM) using MTT assay. Compound 4h (4207) and 4f (4295) are protective against the effect of lactacystin in this model; and
Figure 6 illustrates the viability of ventral mesencephalic neurones using calcein AM assay. Low to medium concentrations of compound 4h (4207) and 4f (4295) reversed cell death in response to 5 µM lactacystin.

### Examples (Only examples covered by the scope of the claims form part of the claimed invention)

### Synthesis and characterization of N-arylpiperazine derivatives

### Overview

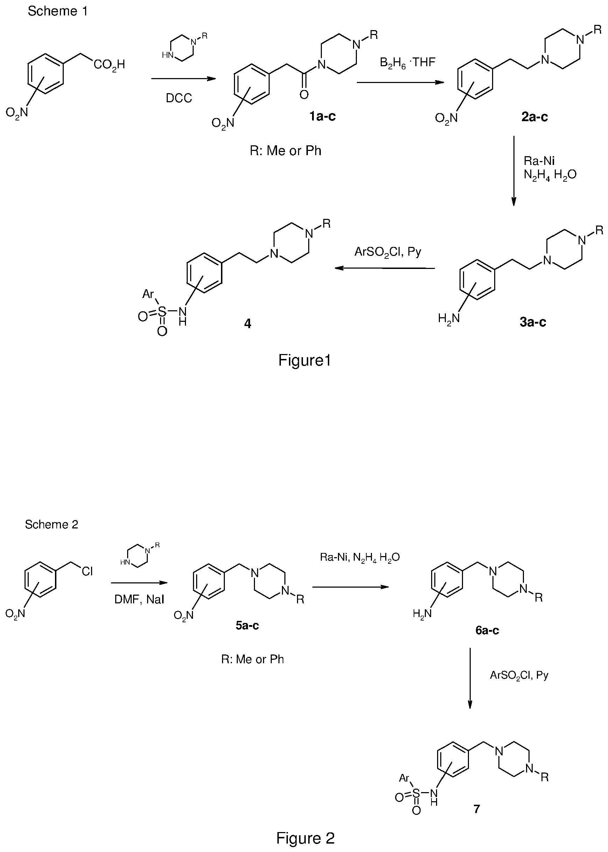

Synthetic route and chemical structures of the compounds synthesized in the present study are shown in Figure 1 and Figure 2. Acylation of N-phenylpiperazine using nitrophenylacetic acid gave rise to 2-(nitrophenyl)-1-(4-phenyl-piperazin-1-yl)-ethanones (1a-c). Amides 1a-c were converted to 1-nitro-phenethyl-4-phenyl-piperazines 2a-c using diborane in tetrahydrofuran (THF). Nitro compounds 5a-c were obtained by direct alkylation of arylpiperazines with corresponding nitrobenzyl halides (Figure 2). Reduction of nitro compounds 2a-c and 5a-c by Ra-Ni/hydrazine provided anilines 3a-c and 6a-c. Target compounds 4a-c and 7a-c were obtained by condensation of anilines 3a-c and 6a-c with corresponding aromatic sulphonyl chloride in presence of pyridine in N-Dimethylformamide (DMF). All compounds were characterized by NMR spectroscopy and mass spectroscopy.

### General

A Stuart SMP10 apparatus (Bibby Sterlin Ltd, Stone, UK) was used to determine melting points, presented here as uncorrected. 1H-NMR (at 300 MHz) and 13C-NMR (at 50 MHz) spectra were recorded on a Bruker AC300 (Bruker, Karlsruhe, Germany) with CDCl3 as a solvent, and, unless otherwise stated, are reported in ppm using tetramethylsilane as the internal standard. The mass spectra were determined by field desorption mass spectroscopy on a Finnigan Mat 8230 mass spectrometer (Finnigan, Bremen, Germany). For analytical thin-layer chromatography Merck (Darmstadt, Germany) F-256 plastic-backed thin-layer silica gel plates were used. Chromatographic purifications were performed on Merck-60 silica gel columns, 230-400 mesh ASTM, under medium pressure (dry column flash chromatography). All reagents and solvents used herein were obtained from Aldrich and were used without further purification. Solutions were routinely dried over anhydrous MgSO4 prior to evaporation.

A library of several hundred new arylpiperazines has been synthesized and screened for neuroprotective activity. Synthesis arylpiperazines containing heteroaryl sulphonamide fragments as a part of molecule is shown in Figures 1 and 2.

### Example 1:

### 2-(Nitro-phenyl)-1-(4-phenyl-piperazin-1-yl)-ethanones (1a-c):

1-Phenylpiperazine (10.75 g, 10.12 mL, 66.24 mmol), *o-, m*- or *p*-nitrophenylacetic acid (12 g, 66.24 mmol) and triethylamine (7.37g, 10.14 mL, 72.87 mmol) were dissolved in 200 ml DMF dry. The obtained solution was cooled to 10°C with an ice bath, a PPAA solution (72.70 mmol, 42.5 mL, 50% solution in DMF) was dropwise added and the reaction mixture (orange solution) was left with stirring, at RT overnight. The reaction mixture was poured onto ice/water. The pH of the suspension was adjusted to 8 with 10% Na₂CO₃ solution (from acidic range). The precipitate was filtrated, washed with water and crystallized from acetone to give the expected product.
Yield: 2-(2-Nitro-phenyl)-1-(4-phenyl-piperazin-1-yl)-ethanone (1a): 17.2 g white crystals; mp 140-141 °C, 2-(3-Nitro-phenyl)-1-(4-phenyl-piperazin-1-yl)-ethanone (1b): 12.5 g; white crystals; mp 124-126 ºC and 2-(4-Nitro-phenyl)-1-(4-phenyl-piperazin-1-yl)-ethanone (1c): 19.0 g; white crystals; mp 147-150 ºC.

### Example 2:

### 1-[2-(Nitro-phenyl)-ethyl]-4-phenyl-piperazines (2a-c):

To a suspension of 2-(2-nitro-phenyl)-1-(4-phenyl-piperazin-1-yl)-ethanone (1a) or 2-(3-nitro-phenyl)-1-(4-phenyl-piperazin-1-yl)-ethanone (2a) or 2-(4-nitro-phenyl)-1-(4-phenyl-piperazin-1-yl)-ethanone (3a) (15 g, 46.1 mmol) in 300 ml dry THF, a diborane solution (1 M in THF, 118 mL, 118 mmol) was added dropwise while cooling externally in an ice-water bath. The ice bath was removed after the addition of diborane was completed and the reaction mixture was heated with a heat-gun to seeding. After spontaneous boiling stops the reaction mixture was refluxed for an additional 2 h. After cooling down to RT water (35 ml) was added drop wise followed by 5.5 M HCl (70 ml). The reaction mixture was refluxed for another 60 min and was left to cool down to RT. The reaction mixture was concentrated under vacuum and the resulting solution was treated with 10% NaHCO₃ solution to pH∼8. The product was extracted with ethyl acetate, washed with water and brine and evaporated to dryness to give yellow oil. Trituration with hexane gave the product.
Yield: 1-[2-(2-Nitrophenyl)-ethyl]-4-phenyl-piperazine (2a): 12.7 g yellow powder; mp 74-75°C, 1-[2-(3-Nitrophenyl)-ethyl]-4-phenyl-piperazine (2b): Yield: 9.5 g; white crystals; mp 88-90 ºC and 1-[2-(4-Nitrophenyl)-ethyl]-4-phenyl-piperazine (2c): 14.0 g; white crystals; mp 137-140 ºC.

### Example 3:

### 1-[2-(Nitro-phenyl)-methyl]-4-phenyl-piperazines (5a-c):

1-Phenylpiperazine (10.75 g, 10.12 mL, 66.24 mmol), *o-, m*- or *p*-nitrobenzyl chloride (11.3 g, 66.24 mmol) and sodium carbonate (6.93g, 66.24 mmol) were resuspend in 100 ml DMF dry. The obtained solution was brought to 60 °C and left with stirring overnight. The reaction mixture was poured onto ice/water. The precipitate was filtrated, washed with water and crystallized from ethanol to give the expected product.
Yield: 1-[2-(2-Nitrophenyl)-methyl]-4-phenyl-piperazine (6a): 15.7 g yellow powder; mp 102-105°C, 1-[2-(3-Nitrophenyl)-methyl]-4-phenyl-piperazine (6b): Yield: 16.5 g; white crystals; mp 128-130 ºC and 1-[2-(4-Nitrophenyl)-methyl]-4-phenyl-piperazine (6c): 18.0 g; white crystals; mp 169-170 ºC.

### Example 4:

### [2-(4-Phenyl-piperazin-1-yl)-ethyl]-phenylamines (3a-c) and [2-(4-Phenyl-piperazin-1-yl)-methyl]-phenylamines (6a-c):

To a ethanol solution of 1-[2-(2-Nitrophenyl)-ethyl]-4-phenyl-piperazine (2a) or 1-[2-(3-Nitrophenyl)-ethyl]-4-phenyl-piperazine (2b) or 1-[2-(4-Nitrophenyl)-ethyl]-4-phenyl-piperazine (2c) (110 ml, 3 g, 9.36 mmol) and hydrazine hydrate (5.0 ml) were successively added, Ra/Ni (0.5-0.7 g). The reaction mixture was stirred at rt for 3 h and for 1 h at 50 °C. The suspension was filtrated over a bed of Celite and the filtrate was evaporated in a vacuum. The residue was taken in ethyl acetate and washed with water/bicarbonate solution, water and brine. After being evaporated the oily residue was crystallized from ethyl acetate.
Yield: 2-[2-(4-Phenyl-piperazin-1-yl)-ethyl]-phenylamine (3a): 2.53g , pink crystals; mp 101-103°C; 3-[2-(4-Phenyl-piperazin-1-yl)-ethyl]-phenylamine (3b): 2.3 g; mp 105-108 ºC; white crystals; 4-[2-(4-Phenyl-piperazin-1-yl)-ethyl]-phenylamine (3c): 2.8 g; white crystals; mp 202-205 ºC 2-[2-(4-Phenyl-piperazin-1-yl)-methyl]-phenylamine (6a): 2.23g , pink crystals; mp 131-134°C; 3-[2-(4-Phenyl-piperazin-1-yl)-methyl]-phenylamine (6b): 2.13 g; mp 172-175 ºC; white crystals; and 4-[2-(4-Phenyl-piperazin-1-yl)-methyl]-phenylamine (6c): 2.38 g; white crystals; mp 223-224 ºC.

### Example 5:

### N-{2-[2-(4-phenyl-piperazin-1-yl)-ethyl]-phenyl}-2-pyridylsulphonamide (4d):

2-Pyridinesulfonyl chloride, *0.37 g* (2.2 mmol), was added to the solution of 560 mg of 2-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from methanol to give 480 mg of product, mp: 233-235°C.
¹H NMR (300 MHz, *DMSO*-*d₆*) δ ppm: 10.15 (brs, 1H, NH), 8.82 (ddd, *J* = 4.7 Hz, *J* =1.7 Hz, *J =*0.9 Hz, 1H, Ar-H), 8.06 (td, *J =* 7.8 Hz, *J =* 1.7 Hz, 1H, Ar-H), 7.87 (dt, *J* = 7.9 Hz, *J =* 0.9 Hz, 1H, Ar-H), 7.70 (ddd, *J =* 7.7 Hz, *J =*4.7 Hz, *J =* 1.1 Hz, 1H, Ar-H), 7.34-7.25 (m, 3H, Ar-H), 7.19 (td, *J* = 7.5 Hz, *J* = 1.4 Hz, 1H, Ar-H), 7.12 (td, *J* = 7.5 Hz, *J* = 1.7 Hz, 1H, Ar-H), 7.03 (d, *J* = 7.9 Hz, 2H, Ar-H), 6.91 (dd, *J* = 7.9 Hz, *J* = 1.3 Hz, 1H, Ar-H), 6.87 (t, *J =* 7.3 Hz, 1H, Ar-H), 3.90-3.80 (m, 2H, -CH₂-), 3.65-3.55 (m, 2H, -CH₂-), 3.34-3.29 (m, 4H, -CH₂-), 3.18-3.13 (m, 4H, -CH₂-); ¹³C NMR (100 MHz, *DMSO- d₆*) δ ppm:156.88, 150.18, 149.49, 138.76,134.54, 133.85, 130.17, 129.06, 127.42, 127.36, 126.95, 126.85, 124.53, 122.33, 119.89, 115.86, 55.10, 50.54, 45.27, 24.66; HR-MS: *m*/*z* [M+H]⁺ calcd. for C₂₃H₂₇N₄O₂S: 423.1855, found 423.1845.

### Example 6:

### N-{3-[2-(4-phenyl-piperazin-1-yl)-ethyl]-phenyl)-2-pyridylsulphonamide (4e):

2-Pyridinesulfonyl chloride (prepared according to the method described in US2007/60623), *0.37 g* (2.2 mmol), was added to the solution of 560 mg of 3-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from methanol to give 460 mg of product, mp: 210-212°C.
¹H NMR (300 MHz, *DMSO- d₆*) δ ppm: 10.63 (s, 1H, NH), 8.72 (ddd, *J =* 4.6 Hz, *J =*1.6 Hz, *J =*0.9 Hz, 1H, Ar-H), 8.06 (td, *J =* 7.6 Hz, *J =* 1.7 Hz, 1H, Ar-H), 7.99 (dt, J *=* 8.1 Hz, *J =* 1.1 Hz, 1H, Ar-H), 7.640 (ddd, *J =* 7.3 Hz, *J =*4.7 Hz, *J =* 1.3 Hz, 1H, Ar-H), 7.26 (dd, *J =* 8.5 Hz, *J =* 7.4 Hz, 2H, Ar-H), 7.18 (t, *J* = 7.8 Hz, 1H, Ar-H), 7.04 (brs, 1H, Ar-H), 7.03-6.99 (m, 3H, Ar-H), 6.94 (d, *J* = 7.6 Hz, 1H, Ar-H), 6.86 (t, *J =* 7.2 Hz, 1H, Ar-H), 3.83-3.80 (m, 2H, -CH₂-), 3.62-3.59 (m, 2H, -CH₂-), 3.25-3.14 (m, 6H, -CH₂-), 3.06-3.00 (m, 2H, -CH₂-); ¹³C NMR (100 MHz, *DMSO*-*d₆* δ ppm: 156.47, 150.29, 149.71, 138.87, 138.18, 138.04, 129.44, 129.24, 127.54, 124.27, 122.66, 120.41, 120.09, 118.45, 116.08, 55.89, 50.64, 45.50, 29.31; FD-MS: *m*/*z* (%): 422.7 [M]⁺ (100); HR-MS: *m*/*z* [M+H]⁺ calcd. for C₂₃H₂₇N₄O₂S: 423.1855, found 423.1867.

### Example 7:

### N-{4-[2-(4-phenyl-piperazin-1-yl)-ethyl]-phenyl}-2-pyridylsulphonamide (4f):

2-Pyridinesulfonyl chloride, *0.37 g* (2.2 mmol), was added to the solution of 560 mg of 4-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from methanol to give 520 mg of product, mp: 125-127°C.
¹H NMR (300 MHz, *DMSO- d₆*) δ ppm: 10.58 (s, 1H, NH), 8.71 (ddd, *J =* 4.8 Hz, *J* =1.7 Hz, *J =*0.9 Hz, 1H, Ar-H), 8.05 (td, *J =* 7.7 Hz, *J =* 1.7 Hz, 1H, Ar-H), 7.96 (dt, *J =* 7.9 Hz, *J =* 1.0 Hz, 1H, Ar-H), 7.64 (ddd, *J =* 7.4 Hz, *J =*4.7 Hz, *J =* 1.2 Hz, 1H, Ar-H), 7.25 (dd, *J =* 8.6 Hz, *J =* 7.3 Hz, 2H, Ar-H), 7.12-7.11 (m, 4H, Ar-H), 6.99 (d, *J* = 7.9 Hz, 2H, Ar-H), 6.85 (t, *J =* 7.3 Hz, 1H, Ar-H), 3.82-3.78 (m, 2H, -CH₂-), 3.59-3.56 (m, 2H, -CH₂-), 3.30-3.24 (m, 2H, -CH₂-), 3.19-3.09 (m, 4H, -CH₂-), 3.02-2.97 (m, 2H, -CH₂-); ¹³C NMR (100 MHz, *DMSO- d₆*) δ ppm: 156.35, 150.06, 149.50, 38.68, 136.16, 132.54, 129.20, 129.02, 127.31, 122.37, 120.32, 119.88, 115.87, 55.80, 50.46, 45.26, 28.39; FD-MS: *m*/*z* (%): 422.6 [M]⁺ (100); HR-MS: *m*/*z* [M+H]⁺ calcd. for C₂₃H₂₇N₄O₂S: 423.1855, found 423.1867.

### Example 8:

### N-{2-[2-(4-phenyl-piperazin-1-yl)-ethyl]-phenyl}-3-pyridylsulphonamide hydrochloride (4g):

3-Pyridinesulfonyl chloride (prepared according to the method described in US2006/217387 A1), *0.37 g* (2.2 mmol), was added to the solution of 560 mg of 2-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from dilute ethanol as hydrochloride salt, yield was 530 mg of product, mp: 175-176°.
¹H NMR (300 MHz, *DMSO- d₆*) δ ppm: 8.80-8.78 (m, 2H, Ar-H), 8.05 (ddd, *J =* 8.1 Hz, *J =*2.3 Hz, *J =*1.6 Hz, 1H, Ar-H), 7.59 (ddd, *J =* 8.0 Hz, *J =*4.8 Hz, *J =* 0.7 Hz, 1H, Ar-H), 7.22 (dd, *J =* 8.7 Hz, *J =* 7.3 Hz, 2H, Ar-H), 7.17 (dd, *J =* 7.2 Hz, *J =* 1.2 Hz, 1H, Ar-H), 7.13-7.01 (m, 3H, Ar-H), 6.96 (d, *J* = 7.9 Hz, 2H, Ar-H), 6.79 (t, *J =* 7.3 Hz, 1H, Ar-H), 3.30-3.27 (m, 4H, -CH₂-), 2.72-2.69 (m, 4H, -CH₂-), 2.61-2.56 (m, 4H, -CH₂-); ¹³C NMR (100 MHz, *DMSO- d₆*) δ ppm:153.03, 150.69, 146.76, 137.20, 136.00, 135.38, 134.35, 130.70, 128.87, 127.00, 125.10, 124.28, 123.66, 118.86, 115.37, 58.56, 52.39, 47.37, 28.77; ESI-MS: *m*/*z* (%): 423.22 [M+H]⁺ (100); HR-MS: *m*/*z* [M+H]⁺ calcd. for C₂₃H₂₇N₄O₂S: 423.1855, found 423.1873.

### Example 9:

### N-{3-[2-(4-phenyl-piperazin-1-yl)-ethyl]-phenyl}-3-pyridylsulphonamide hydrochloride (4h):

3-Pyridinesulfonyl chloride, *0.37 g* (2.2 mmol), was added to the solution of 560 mg of 3-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from dilute ethanol as hydrochloride salt, yield was 590 mg of product, mp: 158-159°C.
¹H NMR (300 MHz, *DMSO- d₆*) δ ppm: 8.87 (dd, *J =* 2.4 Hz, *J =*0.7 Hz, 1H, Ar-H), 8.77 (dd, *J =* 4.8 Hz, *J =*1.6 Hz, 1H, Ar-H), 8.12 (ddd, *J =* 8.1 Hz, *J =*2.4 Hz, *J =*1.6 Hz, 1H, Ar-H), 7.59 (ddd, *J =* 8.1 Hz, *J =*4.8 Hz, *J =* 0.8 Hz, 1H, Ar-H), 7.24 (dd, *J =* 8.7 Hz, *J =* 7.3 Hz, 2H, Ar-H), 7.20 (t, *J* = 7.8 Hz, 1H, Ar-H), 7.04 (t, *J* = 1.6 Hz, 1H, Ar-H), 7.00-6.93 (m, 4H, Ar-H), 6.83 (t, *J =* 7.3 Hz, 1H, Ar-H), 3.40-3.33 (brs, 4H, -CH₂-), 3.26-2.20 (m, 4H, -CH₂-), 3.15-3.10 (m, 2H, -CH₂-), 2.93-2.87 (m, 2H, -CH₂-); ¹³C NMR (100 MHz, *DMSO*-*d₆*) δ ppm:163.00, 153.48, 149.74, 147.03, 138.85, 137.27, 135.65, 134.71, 129.51, 129.03, 124.98, 124.31, 120.73, 119.74, 118.80, 115.80, 56.36, 50.93, 45.87, 29.79; FD-MS: *m*/*z* (%): 422.5 [M]⁺ (100); HR-MS: *m*/*z* [M+H]⁺ calcd. for C₂₃H₂₇N₄O₂S: 423.1855, found 423.1873.

### Example 10:

### N-{4-[2-(4-phenyl-piperazin-1-yl)-ethyl]-phenyl}-3-pyridylsulphonamide hydrochloride (4i):

3-Pyridinesulfonyl chloride, *0.37 g* (2.2 mmol), was added to the solution of 560 mg of 4-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from dilute ethanol as hydrochloride salt, yield was 620 mg of product, mp: 177-178°C.
¹H NMR (300 MHz, *DMSO- d₆*) δ ppm: 8.84 (dd, *J =* 2.4 Hz, *J =*0.7 Hz, 1H, Ar-H), 8.76 (dd, *J =* 4.8 Hz, *J =*1.6 Hz, 1H, Ar-H), 8.09 (ddd, *J =* 8.1 Hz, *J =*2.4 Hz, *J =*1.6 Hz, 1H, Ar-H), 7.59 (ddd, *J =* 8.1 Hz, *J =*4.8 Hz, *J =* 0.8 Hz, 1H, Ar-H), 7.19 (dd, *J =* 8.7 Hz, *J =* 7.3 Hz, 2H, Ar-H), 7.13 (d, *J* = 8.5 Hz, 2H, Ar-H), 7.00 (d, *J* = 8.5 Hz, 2H, Ar-H), 6.92 (d, *J* = 7.9 Hz, 2H, Ar-H), 6.76 (t, *J =* 7.2 Hz, 1H, Ar-H), 3.13-3.09 (brs, 4H, -CH₂-), 2.71-2.66 (m, 2H, -CH₂-), 2.60-2.53 (m, 6H, -CH₂-); ¹³C NMR (100 MHz, *DMSO- d₆*) δ ppm:153.37, 150.85, 146.99, 135.74, 134.78, 134.63, 129.46, 128.85, 124.28, 120.97, 118.78, 115.29, 59.10, 52.41, 47.88, 31.69; FD-MS: *m*/*z* (%): 422.5 [M]⁺ (100); HR-MS: *m*/*z* [M+H]⁺ calcd. for C₂₃H₂₇N₄O₂S: 423.1855, found 423.1840.

### Example 11: (Reference)

### N-{2-[2-(4-phenyl-piperazin-1-yl)-methyl]-phenyl}-2-pyridylsulphonamide (7d):

2-Pyridinesulfonyl chloride, *0.37 g* (2.2 mmol), was added to the solution of 530 mg of 2-[2-(4-phenyl-piperazin-1-yl)-methyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from methanol to give 580 mg of product, mp: 225-226°C.
¹H NMR (300 MHz, *DMSO- d₆*) δ ppm: 11.37 (brs, 1H, NH), 10.44 (brs, 1H, NH), 8.80 (ddd, *J =* 4.7 Hz, *J =* 1.6 Hz, *J =* 0.8 Hz, 1H, Ar-H), 8.07 (td, *J =* 7.8 Hz, J *=*1.7 Hz, 1H, Ar-H), 7.91 (dt, *J =* 7.9 Hz, *J =* 0.8 Hz, 1H, Ar-H), 7.85 (dd, *J =* 7.3 Hz, *J =* 1.7 Hz, 1H, Ar-H), 7.70 (ddd, *J =* 7.6 Hz, *J =* 4.7 Hz, *J =* 1.1 Hz, 1H, Ar-H), 7.37-7.31 (m, 3H, Ar-H), 7.26 (dd, *J =* 8.4 Hz, *J =* 7.5 Hz, 2H, Ar-H), 7.15 (dd, *J =* 7.5 Hz, *J* = 1.8 Hz, 1H, Ar-H), 6.98 (d, *J =* 8.0 Hz, 2H, Ar-H), 6.86 (t, *J =* 7.3 Hz, 1H, Ar-H), 4.48 (s, 2H, -CH₂-), 3.80-3.77 (brs, 2H, -CH₂-), 3.44-3.20 (brs, 6H, -CH₂-); ¹³C NMR (100 MHz, *DMSO-d₆*) δ ppm:156.61, 150.15, 149.37, 138.91, 136.23, 133.06, 130.38, 129.07, 127.53, 127.04, 126.85, 125.80, 122.38, 119.90, 115.86, 54.11, 50.37, 45.22; FD-MS: *m*/*z* (%): 408.6 [M]⁺ (100); HR-MS: *m*/*z* [M+H]⁺ calcd. for C₂₂H₂₅N₄O₂S: 409.1698, found 409.1685.

### Example 12: (Reference)

### N-{3-[2-(4-phenyl-piperazin-1-yl)-methyl]-phenyl}-2-pyridylsulphonamide (7e):

2-Pyridinesulfonyl chloride, *0.37 g* (2.2 mmol), was added to the solution of 530 mg of 3-[2-(4-phenyl-piperazin-1-yl)-methyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from methanol to give 590 mg of product, mp: 228-231 °C.
¹H NMR (300 MHz, *DMSO-d₆*) δ ppm: 10.51 (s, 1H, NH), 8.71 (ddd, *J =* 4.7 Hz, *J =* 1.7 Hz, *J =* 0.9 Hz, 1H, Ar-H), 8.04 (td, *J =* 7.7 Hz, *J =*1.7 Hz, 1H, Ar-H), 7.94 (dt, *J =* 7.9 Hz, *J =* 1.0 Hz, 1H, Ar-H), 7.63 (ddd, *J =* 7.6 Hz, *J =* 4.7 Hz, *J =* 1.2 Hz, 1H, Ar-H), 7.20 (dd, *J =* 8.7 Hz, *J =* 7.3 Hz, 2H, Ar-H), 7.15 (t, *J =* 7.7 Hz, 1H, Ar-H), 7.10 (t, *J* = 1.6 Hz, 1H, Ar-H), 7.03 (ddd, *J =* 8.0 Hz, *J =* 2.1 Hz, *J =* 1.0 Hz, 1H, Ar-H), 6.96-6.91 (m, 3H, Ar-H), 6.76 (t, *J =* 7.2 Hz, 1H, Ar-H), 3.39 (s, 2H, -CH₂-), 3.08-3.05 (brs, 4H, -CH₂-), 2.38-2.35 (brs, 4H, -CH₂-); ¹³C NMR (100 MHz, *DMSO- d₆*) δ ppm:156.12, 150.13, 149.39, 138.69, 137.88, 130.31, 129.45, 129.04, 127.39, 127.08, 122.93, 122.55, 121.19, 119.84, 115.86, 57.91, 49.86, 45.02; FD-MS: *m*/*z* (%): 408.6 [M]⁺ (100); HR-MS: *m*/*z* [M+H]⁺ calcd. for C₂₂H₂₅N₄O₂S: 409.1698, found 409.1708.

### Example 13: (Reference)

### N-{4-[2-(4-phenyl-piperazin-1-yl)-methyl]-phenyl}-2-pyridylsulphonamide (7f):

2-Pyridinesulfonyl chloride, *0.37 g* (2.2 mmol), was added to the solution of 530 mg of 4-[2-(4-phenyl-piperazin-1-yl)-methyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from methanol to give 670 mg of product, mp: 178-179°C.
¹H NMR (300 MHz, *DMSO- d₆*) δ ppm: 10.86 (s, 1H, NH), 8.72 (ddd, *J =* 4.9 Hz, *J =* 1.6 Hz, *J =* 0.9 Hz, 1H, Ar-H), 8.04 (ddd, *J =* 7.9 Hz, *J =*1.5 Hz, *J =*1.0 Hz, 1H, Ar-H), 7.66 (ddd, *J =* 7.0 Hz, *J =*4.7 Hz, *J =* 1.6 Hz, 1H, Ar-H), 7.51 (d, *J* = 8.6 Hz, 2H, Ar-H), 7.24 (dd, *J =* 8.6 Hz, *J =* 7.3 Hz, 2H, Ar-H), 7.21 (d, *J* = 8.6 Hz, 2H, Ar-H), 6.94 (d, *J* = 7.9 Hz, 2H, Ar-H), 6.84 (t, *J =* 7.3 Hz, 1H, Ar-H), 4.23 (s, 2H, -CH₂-), 3.77-3.73 (m, 2H, -CH₂-), 3.28-3.10 (m, 6H, -CH₂-); ¹³C NMR (100 MHz, *DMSO- d₆*) δ ppm:156.32, 150.16, 149.42, 138.84, 138.76, 132.33, 129.03, 127.49, 124.47, 122.37, 119.81, 119.11, 115.83, 57.71, 49.89, 45.06; FD-MS: *m*/*z* (%): 408.6 [M]⁺ (100); HR-MS: *m*/*z* [M+H]⁺ calcd. for C₂₂H₂₅N₄O₂S: 409.1698, found 409.1689.

### Example 14: (Reference)

### N-{2-[2-(4-phenyl-piperazin-1-yl)-methyl]-phenyl}-3-pyridylsulphonamide hydrochloride (7g):

3-Pyridinesulfonyl chloride, *0.37 g* (2.2 mmol), was added to the solution of 530 mg of 2-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from dilute ethanol as hydrochloride salt, yield was 590 mg of product, mp: 222-224°C
¹H NMR (300 MHz, *DMSO- d₆*) δ ppm: 11.00 (brs, 1H, NH), 10.49 (brs, 1H, NH), 8.99 (d, *J =*1.9 Hz, 1H, Ar-H), 8.87 (m, 3H, Ar-H), 8.61 (ddd, *J =* 8.0 Hz, *J =* 1.9 Hz, *J =* 1.5 Hz, 1H, Ar-H), 8.14 (ddd, *J =* 8.1 Hz, *J =* 2.4 Hz, *J =* 1.6 Hz, 1H, Ar-H), 7.98 (ddd, *J =* 8.0 Hz, *J =* 5.6 Hz, *J =* 0.7 Hz, 1H, Ar-H), 7.86-7.83 (m, 1H, Ar-H), 7.66 (ddd, *J =* 8.1 Hz, *J =* 4.9 Hz, *J =* 0.8 Hz, 1H, Ar-H), 7.39-7.36 (m, 2H, Ar-H), 7.25 (dd, *J =* 8.6 Hz, *J* = 7.4 Hz, 2H, Ar-H), 6.97 (dd, *J =* 8.7 Hz, *J* = 0.7 Hz, 2H, Ar-H), 6.95-6.92 (m, 1H, Ar-H), 6.85 (t, *J =* 7.3 Hz, 1H, Ar-H), 4.33 (s, 2H, -CH₂-), 3.85-3.70 (brs, 2H, -CH₂-), 3.40-3.10 (brs, 6H, -CH₂-); ¹³C NMR (100 MHz, *DMSO- d₆*) δ ppm:153.59, 149.35, 147.09, 146.25, 143.23, 141.31, 139.93, 135.86, 13579, 134.94, 133.24, 130.59, 129.08, 127.27, 126.88, 126.67, 126.13, 124.36, 119.94, 115.89, 53.85, 50.43, 45.23; FD-MS: *m*/*z* (%): 408.5 [M]⁺ (100); HR-MS: *m*/*z* [M+H]⁺ calcd. for C₂₂H₂₅N₄O₂S: 409.1698, found 409.1712.

### Example 15: (Reference)

### N-{3-[2-(4-phenyl-piperazin-1-yl)-methyl]-phenyl}-3-pyridylsulphonamide hydrochloride (7h):

3-Pyridinesulfonyl chloride, *0.37 g* (2.2 mmol), was added to the solution of 530 mg of 3-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from dilute ethanol as hydrochloride salt, yield was 590 mg of product, mp: 166-168°C.
¹H NMR (300 MHz, *DMSO- d₆*) δ ppm: 10.43 (s, 1H, NH), 8.84 (dd, *J =* 2.4 Hz, *J =* 0.8 Hz, 1H, Ar-H), 8.78 (dd, *J =* 4.8 Hz, *J =*1.6 Hz, 1H, Ar-H), 8.07 (ddd, *J =* 8.1 Hz, *J =* 2.4 Hz, *J =* 1.6 Hz, 1H, Ar-H), 7.59 (ddd, *J =* 8.1 Hz, *J =* 4.8 Hz, *J =* 0.8 Hz, 1H, Ar-H), 7.24-7.31 (m, 3H, Ar-H), 7.08 (brt, *J* = 1.6 Hz, 1H, Ar-H), 7.04-7.00 (m, 2H, Ar-H), 6.92 (dd, *J =* 8.8 Hz, *J =* 0.9 Hz, 2H, Ar-H), 6.77 (t, *J =* 7.2 Hz, 1H, Ar-H), 3.41 (s, 2H, -CH₂-), 3.08-3.05 (brs, 4H, -CH₂-), 2.38-2.35 (brs, 4H, -CH₂-); ¹³C NMR (100 MHz, *DMSO- d₆*) δ ppm:153.39, 150.91, 147.02, 139.36, 136.96, 135.50, 134.63, 129.05, 128.82, 125.26, 124.21, 120.85, 119.62, 118.71, 115.28, 61.49, 52.29, 48.07; FD-MS: *m*/*z* (%): 408.6 [M]⁺ (100); HR-MS: *m*/*z* [M+H]⁺ calcd. for C₂₂H₂₅N₄O₂S: 409.1698, found 409.1694.

### Example 16: (Reference)

### N-{4-[2-(4-phenyl-piperazin-1-yl)-methyl]-phenyl}-3-pyridylsulphonamide hydrochloride (7i):

3-Pyridinesulfonyl chloride, *0.37 g* (2.2 mmol), was added to the solution of 530 mg of 4-[2-(4-phenyl-piperazin-1-yl)-ethyl]-phenylamine (2.0 mmol) in 5.0 ml dry pyridine, reaction mixture was stirred at room temperature for 2 h, whereupon diluted with 100 ml ethyl acetate and extracted 2 times with 8% NaHCO₃ (50 ml) and H₂O (50 ml), each. Organic phase was dried over MgSO₄, filtered and concentrated *in vacuo.* Obtained products ware purified by recrystallized from dilute ethanol as hydrochloride salt, yield was 610 mg of product, mp: 177-179°C.
¹H NMR (300 MHz, *DMSO- d₆*) δ ppm: 10.46 (s, 1H, NH), 8.86 (dd, *J =* 7.2 Hz, *J =* 0.5 Hz, 1H, Ar-H), 8.77 (dd, *J =* 4.8 Hz, *J =*1.6 Hz, 1H, Ar-H), 8.10 (ddd, *J =* 8.1 Hz, *J =* 2.2 Hz, *J =* 1.7 Hz, 1H, Ar-H), 7.59 (ddd, *J =* 8.1 Hz, *J =* 4.8 Hz, *J =* 0.6 Hz, 1H, Ar-H), 7.21 (d, *J* = 8.5 Hz, 2H, Ar-H), 7.18 (dd, *J* = 8.6 Hz, *J* = 7.4 Hz, 2H, Ar-H), 7.06 (d, *J* = 8.5 Hz, 2H, Ar-H), 6.89 (d, *J* = 7.9 Hz, 2H, Ar-H), 6.75 (t, *J* = 7.2 Hz, 1H, Ar-H), 3.40 (s, 2H, -CH₂-), 3.09-3.06 (brs, 4H, -CH₂-), 2.44-2.41 (brs, 4H, -CH₂-); ¹³C NMR (100 MHz, *DMSO-d₆*) δ ppm:153.37, 150.91, 146.96, 135.77, 134.60, 134.43, 129.74, 128.79, 124.25, 120.60, 118.66, 115.25, 61.24, 52.39, 48.05; ESI-MS: *m*/*z* (%): 409.2 [M+H]⁺ (100); HR-MS: *m*/*z* [M+H]⁺ calcd. for C₂₂H₂₅N₄O₂S: 409.1698, found 409.1683. Among synthesized sulphonamides N-{3-[2-(4-phenyl-piperazin-1-yl)ethyl]-phenyl}-picolinsulphonamide (4e) and N-{3-[2-(4-phenyl-piperazin-1-yl)ethyl]-phenyl}-nicotinsulphonamide (4h) appeared as most interesting candidates for further in vivo investigation.

### Biological Data

In a series of studies it was shown that the newly synthesized N-{4-[2-(4-phenyl-piperazin-1-yl)ethyl]-phenyl}-picolinamide (1) and N-{3-[2-(4-phenyl-piperazin-1-yl)ethyl]-phenyl}-picolinamide (2) with arylpiperazine moiety possessed some activity at dopaminergic D2 receptors (discussed below) as well as being most active in preventing mitochondrial damage and apoptosis induced by nitric oxide (NO) in SH-SY5Y cells (Tovilovic et al., 2012). Similarly these novel arylpiperazines modulated the apoptotic and autophagic responses induced by 6-OHDA in human SH-SY5Y neuron-like cells.

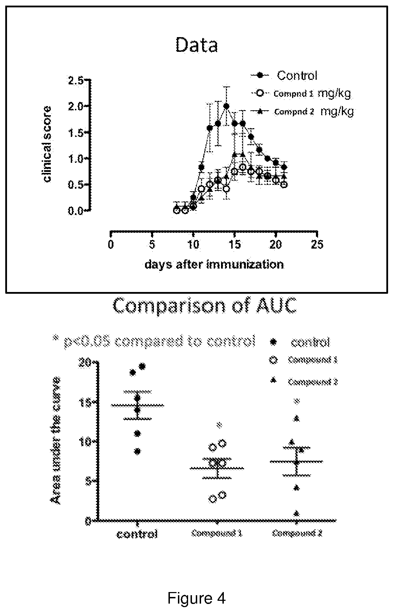

In a parallel field, a number of key studies using rodent models of experimental autoimmune encephalomyelitis (EAE) have demonstrated the potential value of these compounds as potent anti-inflammatory agents. Since in this study it was shown that the 2 compounds significantly reduced motor disability due to neuropathy induced by EAE in vivo, this therefore demonstrates good blood brain barrier penetrability (see Figure 4). Thus a variety of approaches have suggested that these compounds bring about neuroprotective effects that might be of great value in Parkinson's disease.

Figure 4 illustrates the effect of compounds 1 and 2 on motor clinical scores (Rats were evaluated daily and graded by a blinded investigator according to the following scale: grade 0 = no signs; grade 1 = limp tail; grade 2 = hind limb weakness sufficient to impair righting; grade 3 = paraplegia) following IV administration of 70 microgram myelin basic protein suspended in Freund's adjuvant. The top panel compares clinical score against time. In the bottom panel the area under the curves for the duration of the experimental observation is compared.

Finally, it should be noted that newly described arylpiperazine 1 and 2 are a partial dopaminergic agonist with moderate affinity towards dopamine D2 receptors. However, the neuroprotective activity of those two compounds ware apparently independent of dopamine receptor binding (Tovilovic et al., 2012). Chemical proteomics study revealed NAD(P)H dehydrogenase (Quinone) (NQO1) as putative mitochondrial target for these compounds.

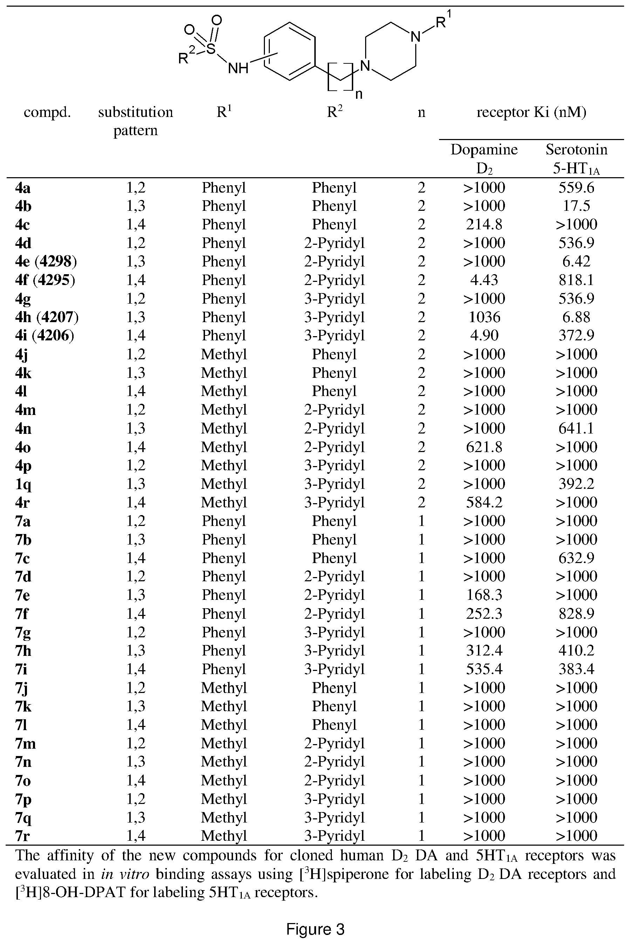

From a range of sulphonsmides synthesized, we have identified 2 interesting compounds: 4298 and 4207, which possess little or no affinity for dopamine D2 receptors but high affinity for 5-HT1a receptors (Ki values: 6.42 and 6.88 nM respectively, see Figure 3). The rationale for choosing these compounds is that there would be little if any confounding effects due to activation or antagonism of dopamine receptors. These compounds will have interesting characteristics that would make them suitable for further investigation in neuroprotection investigations as well as in modulating the symptoms of neurological and neuropsychiatric diseases.

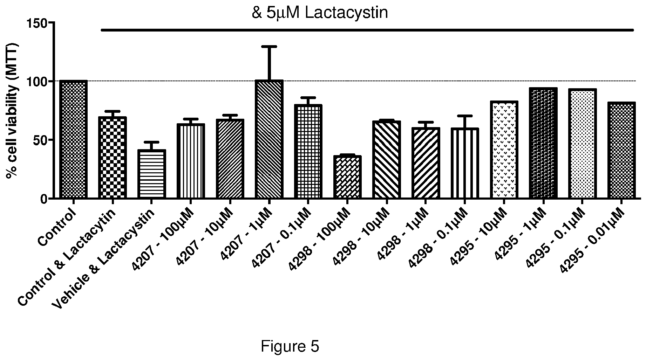

Compounds 4298 and 4208 as well as 4295 (which had high affinity for D2 receptor: Ki value=4.43 nM) were tested against lactacystin (5µM) cytotoxicity on rat primary ventral mesencephalic neurones. All compounds markedly protected the dopaminergic neurons against 5µM lactacystin (see Figure 5) in an MTT assay. The compounds 4207 which possessed little or no dopaminergic activity offered most protection against lactacystin.

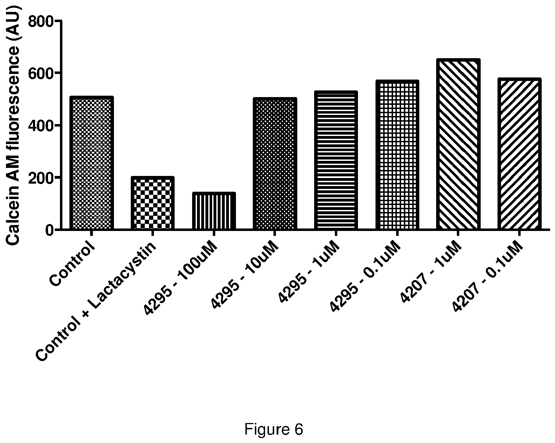

When 4207 and 4295 (no D2 affinity vs. high D2 affinity) were tested against 5 µM lactacystin in calcein AM fluorescence assay, both compounds were able to afford protection at concentrations ranging from 0.1 to 1 µM, suggesting that the protective effect of N-arylpiperazine heteroaryl sulphonamides were not related activation of D2 receptors. However, at higher concentrations ranging from 10 to 100 µM neuroprotection was reversed, indicating a bimodal concentration response relationship (see Figure 6).

The present invention relates to of formula A, I, II or III and pharmaceutically acceptable salts or solvates thereof and, in particular, to their activity as 5-hydroxytryptamine 1A (5-HT1A) receptor subtype ligands, to their stereo chemical isomers and to their use for the treatment of pathological conditions for which the pharmacological properties inherent in these compounds is indicated. In particular, 5-HT1A receptor has been a major target for neurobiological research and drug development due to its implication in many pathophysiological processes. 5-HT1A ligands may find use in the treatment of several diseases such as anxiety, depression, schizophrenia, sexual dysfunction, cognitive deficits resulting from neurodegenerative diseases like Alzheimer's disease, nausea and vomiting, sleep disorders, pain, obesity, pain, addiction, withdrawal and in the treatment of prostate cancer. More recent evidence now indicates that 5-HT1A ligands act in other disease states and conditions by virtue of their ability to inhibit the release of glutamate (Choi et al., 2013). 5-HT1A ligands may be used to treat conditions arising from the dysfunction of the glutamate neurotransmitter system or the aberrant release of glutamate.

Therefore, the present invention relates to medicaments in which the ligands comprising an active ingredient derivative or a salt thereof represented by formula A, I, II or III and pharmaceutically acceptable salts or solvates thereof for treatment of patients suffering from disorders of the central and/or peripheral nervous system in which 5-HT1A and/or dopamine D2 receptor subtype activity play an important role. Thus, ligands incorporating the above chemical moiety might be useful as therapeutic or prophylactic agent for a variety of disorders where a therapeutic role following the binding of the ligands (described in this invention) to 5-HT1A and or D2 receptor has been described.

Brain serotonin (5-HT) plays a major role in a number of physiological processes and pathological conditions. Serotonin neurotransmission is involved in the mood regulation, impulse control, vigilance and sleep, eating, sexual behaviour, and cognitive functions, such as memory and learning (Maes and Meltzer, 1995; Overstreet et al., 1998; King et al., 2008; Newman-Tancredi and Klevan, 2011). In addition, serotonin is important in the modulation of anxiety and fear, as well as impulsiveness in suicidal and other violent acts (Dourish, 1987; Murphy, 1990; Fuller, 1991; Lesch, 1991; Lemonde et al., 2003; Akimova et al., 2009). In the periphery, serotonin plays an important role in the gastro-intestinal functioning (Gershon, 2004, 2005).

Therefore, based on the extensive role serotonin following activation of 5-HT1A receptors (Lucki, 1998), the compounds described in this invention will be useful in the following indications:

### Mood disorders: depression and anxiety

There is considerable evidence for the role of aberrant serotonin transmission in depression and anxiety (Akimova et al., 2009; Blier and Mansari, 2013). In several experimental paradigms ligands activating the 5-HTIA receptor have been shown to be effective in reducing anxiety and depression (Jann, 1998). Therefore, the disclosed compounds, acting through 5-HT1A receptors are likely to be invaluable for the treatment of depressive disorders, including the sub-type disorders major depressive disorder and dysthymic disorder, and for the treatment of generalized anxiety disorders (GAD). In addition, combination treatment with SSRIs and the disclosed compounds might also have beneficial effects in treatment resistant major depressive disorders (Papakostas et al., 2005) by enhancing the therapeutic potential of SSRIs in treatment of depression (Cittrome, 2012).

Furthermore, due to activity profile and similarity with buspirone, a non-benzodiazepine anxiolytic acting on 5-HT1A and D2 receptor, the compounds described in this invention are likely to have anxiolytic properties and will thus be useful for treatment of generalized anxiety disorders.

Recently the presence of a new subtype of depression has been hypothesized: Stressor-precipitated, cortisol-induced, serotonin-related, anxiety/aggression-driven depression. Patients suffering from this subtype of depression, exhibit impaired serotonin synthesis and reduced 5-HT1A receptor sensitivity (van Praag, 1997). As specific therapeutic agents selective 5-HT1A agonists and cortisol or CRH antagonists are proposed. Prophylactically, maintenance treatment with 5-HT1A agonists seems indicated as well as psychological interventions to increase the stressor threshold.

### Bipolar Depression

Aripiprazole (Abilify) is a new antipsychotic undergoing phase III clinical trials for type I bipolar disorder as an adjunct to mood stablizers such lithium or valproate. This agent, which possesses unique capabilities compared to other antipsychotic agents, especially because of its partial dopaminergic agonistic activity. Moreover, like the other atypical agents, aripiprazole is an antagonist of the 5-HT2A receptor, and an agonist of the 5-HT1A receptor. Aripiprazole is well recognized for its capacity to potentiate antidepressants in the treatment of unipolar depression. Furthermore, it is well known that treatment of bipolar estate with mood stabilizers can markedly affect the expression of 5-HT1A receptors (Nugent et al., 2013). The similarity of action profile of the current disclosed compounds suggest a beneficial effect for in this indication either directly of as adjunct to mood stabilizers.

### Social Phobia

Buspirone has been investigated as an alternative therapy to monoamine oxidase inhibitors for treatment of social phobia as it lacks the potentially serious side effects associated with those compounds. One open-label study reported positive findings in a group of patients currently receiving SSRI therapy with the addition of buspirone (30-60 mg/day) (Van Ameringen et al., 1996). Therefore, the disclosed compounds either alone or in combination with SSRI may be efficacious for this indication.

### Obsessive compulsive disorder (OCD)

SSRIs such clomipramine, fluoxetine, sertraline, paroxetine, and fluvoxamine are commonly and successfully used in the treatment of OCD. However, while 50 to 70% of OCD patients respond to one of these medications, their symptoms are rarely eliminated (Stanley and Turner, 1995). In a few cases of intractable OCD combination of sertraline and buspirone resulted in dramatic improvements in OCD treatment (Menkes, 1995). Some trials have also reported successful treatment of OCD (Jenike et al., 1991; Pato et al., 1991) following buspirone treatment. Therefore, it is feasible that combination of 5-HT1A agonist disclosed herein would have enhance the benefits of SSRIs in the treatment of OCD.

### Attention deficit hyperactivity disorder (ADHD)

In many neuropsychiatric syndromes, including ADHD and schizophrenia, functioning of the ascending serotonergic system is impaired. Experimental evidence from isolation reared rats suggest that 5-HT1A receptor binding site densities were significantly reduced by 22% in the prelimbic cortex, and significantly increased by between 10 and 50% in the motor cortex, somatosensory cortex, dentate gyrus and CA fields of the hippocampus (Preece et al., 2004).

### Schizophrenia

Schizophrenia has two components: positive symptoms, which include delusion, hallucination and excitation and negative symptoms, which include social withdrawal and apathy. The commonly used drugs for this indication do not fully address the need for treatment of the negative symptoms of this disorder. Several patent application using animal and clinical studies have suggested that combination of D2 receptor blockade and 5-HT1A activation could markedly reduce both the negative and positive symptoms of schizophrenia (NeuroSearch, WO09095438, 2009; WO1004080, 2010).

Furthermore, long term treatment with commonly used drugs may lead to extrapyramidal side effects such as tardive dystonia/dyskinesia. Clinical trials with buspirone have suggested that tartive dyskinesia may be effectively reduced by 5-HT1A activation (Moss, et al., 1993). Due to the proposed efficacy of the disclosed compounds on the 5-HT1A receptor and affinity for D2 receptors, the disclosed compounds may also be beneficial in targeting the extrapyramidal symptoms such as tardive dyskinesia, acute dystonia.

### Movement disorders: Parkinson's disease and levodopa-induced dyskinesia

It has been shown that methyledioxymethamphetamine (MDMA) can markedly reduce levodopa-induced dyskinesia in a primate model of Parkinson's disease (PD) (Iravani et al., 2003). This effect is brought about by direct stimulation of 5-HT1A receptors (Iravani et al., 2006). Several 5-HT1A agents including sarizotan and tandisparone showed some efficacy in reducing levodopa-induced dyskinesia (Kannari et al., 2002; Goetz et al., 2007). Clinical trials with fetal ventral mesencephalic tissue grafts have shown some successes in the treatment of PD, however it can result in graft-induced dyskinesias (GIDs) similar to that observed following long-term symptomatic treatment of PD patients with levodopa. It is suggested that GIDs occur due to an underlying dysfunction in serotonergic neurotransmission (Politis 2010a-c, 2011a-b). Following the administration of buspirone in transplanted PD patients affected by GID; marked attenuation of GID was reported, indicating buspirone may be effective in the treatment of GIDs.

### Neurogenesis

Serotonin is believed to play an important role in cognition, and serotonin receptors may provide a novel target for future anti-dementia therapeutics. Several lines of evidence suggest that cognitive decline might result from progressive loss hippocampal neurons and induction of neurogenesis might be a valuable avenue to explore in combatting progressive cell loss that occur in cognitive decline (Song et al., 2012). There is evidence that 5-HT1A receptors are involved in the mechanism of neurogenesis (Fuss et al., 2013). Understanding the contributions of adult neurogenesis to hippocampal function will provide new insight into the fundamental aspects of brain plasticity, which can be used to guide therapeutic interventions to replace neural populations damaged by disease or injury (Song et al., 2012). Accordingly, it has been shown that 5-HT1A receptors activation in vitro promotes neurogenesis (Klempin et al., 2010). Also, activation of 5-HT1A receptor by selective 5-HT1A agonist, 8-OH-DPAT increased neurogenesis and survival of late differentiating cells in the dentate gyrus and in the olfactory bulb (Soumier et al., 2010). Patent Number 7,678,808 entitled, "5HT Receptor Mediated Neurogenesis." The patent provided coverage for treating depression with buspirone, a 5HT1A agent, in combination with melatonin, by initiating neurogenesis cascade. Buspirone and melatonin are the two active agents in BCI-952, a compound that was selected by BrainCells' platform and that demonstrated proof-of-concept in a clinical study.

### Neuroprotection: Alzheimer's disease, Parkinson's disease, Huntington's disease and Multiple sclerosis, traumatic brain injury, stroke

In rat primary hippocampal cultures, and in rat in vivo middle cerebral artery occlusion model of stroke, novel 5-HT1A agonists have been shown to possess a neuroprotective effect (Valhondo et al., 2013). It has been proposed the neuroprotective mechanisms of 5-HT1A receptors are thought to be the result of neuronal hyperpolarization via the activation of G protein-coupled inwardly rectifying K+ channels upon binding to both pre- and post-synaptic 5-HT1A receptors. Hyperpolarization results in inhibition of neuron firing and reduction of glutamate release.

Glutamate is the predominant neurotransmitter in the central nervous system and it plays an important role in neuroplasticity. As such, excessive extracellular levels of glutamate (excitotoxicity) have been associated With the pathophysiology of both acute neurodegenerative disorders such as stroke, transient ischemic attack and spinal/brain trauma, as Well as chronic neurodegenerative disorders such as epilepsy, Alzheimer's Disease, amyotrophic lateral sclerosis, Huntington's Disease, Parkinson's Disease, AIDS dementia and retinal diseases. Compounds Which inhibit or attenuate the release of glutamate represent potential neuroprotective agents for the treatment of ischemia resulting from stroke, transient ischemic attack, brain/ spinal trauma and fetal hypoxia (Koroshetz and Moskowitz, 1996).

Activation of 5-HT1A receptors inhibit the excessive release of glutamate (Rylander, 2012). In addition to therapeutic applications in the field of psychiatry, more recent preclinical studies have suggested that 5-HT1A receptor ligands have also pronounced neuroprotective properties. 5-HT1A ligands have been proven to be effective in anxiety and depression. In addition to therapeutic applications in the field of psychiatry, more recent preclinical studies have suggested that 5-HT1A receptor ligands have also pronounced neuroprotective properties.

### Neuroprotection: role of 5-HT1A as an anti-inflammatory agent

Activation of 5-HT1A receptors play a prominent role in modulation of the immune system action (Idova and Davydova, 2010). 5-HT is required for optimal accessory function of monocytes and macrophages, acting through 5-HT1A receptors. The observation 5-HT acting on 5-HT1A receptors inhibits proliferation of T cells in response can be taken as a modulatory role of 5-HT1A receptors on immune response (Mossner et al., 1998). Indeed, 5-HT1A receptors might be involved in the pathogenesis of multiple sclerosis and in experimental autoimmune encephalitis (EAE) models (Pedotto et al., 2001). In our preliminary experiments using a group of compounds disclosed herein, significant reduction in EAE-induced disability was observed in the rat.

### Spinocerebellar ataxia - (Machado-Joseph disease)

Spinocerebellar ataxia are clinically heterogeneous group of disorders. Patients exhibit usually a slowly progressive cerebellar syndrome with various combinations of oculomotor disorders, dysarthria, dysmetria/kinetic tremor, and/or ataxic gait. They can present also with pigmentary retinopathy, extrapyramidal movement disorders (parkinsonism, dyskinesias, dystonia, chorea), pyramidal signs, cortical symptoms (seizures, cognitive impairment/behavioral symptoms), peripheral neuropathy (Manto, 2005). The cerebellum receives a prominent serotonergic innervation, consequently targeting 5-HT1 receptors is likely to be beneficial in the treatment of ataxias. Several studies have evaluated buspirone for ataxia treatment. On mild to moderate ataxia, buspirone, a 5-HT1A agonist displayed efficacy in reducing global ataxia (Lou et al., 1995; Trouillas et al., 1995). Consequently, the compounds disclosed herein are likely to be efficacious in this pathology.

### Sleep disorders

5-HT is involved in the regulation of sleep. Various 5-HT1A agonists such as tandospirone (Suzuki et al., 1995) and buspirone (Wilson et al., 2005) have been efficacious in treatment of insomnia, therefore compounds with similar property might have an important therapeutic value.

### Pain

The serotonergic system is highly involved in the regulation of affective, homeostatic and pain related mechanisms (Milan, 1995; Ossipov et al., 2010). Recently, it has been reported that novel 5-HT1A agonist have antinoceptive property in various rodent pain models (Valhondo et al., 2013). In a clinical study model of trigeminal neuralgia (recurrent, severe shooting pain in the area of trigeminal nerve which can be brought about by light touches such as chewing, talking brushing teeth), 5-HT1A agonists reduced mechanical allodynia (Kanai et al., 2006). It has been suggested that in analgesia, 5-HT1A receptor activation has a differential effect compared opioids: stimulation of peripheral nociceptors initially produces pain as a first-order effect, but also sets out hypoalgesia as a second-order effect which with repeated administration this second-order hypoalgesia counteracts the first-order pain and remarkably develops into increasingly powerful analgesia (Colpaert, 2006). Thus agents acting as 5-HT1A agonist, including those addressed in this disclosure might be beneficial in the treatment of neuropathic pain and in particular as adjunct therapy to opioid analgesics such as tramadol (Berrocoso et al., 2007).

### Age-related macular degeneration

Age-related macular degeneration (AMD) is a painless eye condition that generally leads to the gradual loss of macula (photoreceptor (rods and cones) involved in central vision but can sometimes cause a rapid irreversible vision loss in older age. AMD is the leading cause of visual impairment in the UK, affecting up to 500,000 people to some degree. For reasons that are unclear, AMD tends to be more common in women than men. It is also more common in white people and people of Chinese ethnicity than people from other ethnic groups. Age is one of the most important risk factors for AMD. The condition is most common in people over 50 and it's estimated that one in every 10 people over 65 have some degree of AMD (Sharma et al., 2014). The products of oxidative stress trigger chronic low-grade inflammation (pathophysiological parainflammation) process in AMD patients. Microglial activation induces photoreceptor cells injury and leads to the development of dry AMD (Nita et al., 2014).

In rats, retinal exposure to blue causes severe photo-oxidative damage and consequently can model features of AMD, including photoreceptor and retinal pigment epithelium degeneration, choriocapillaris atrophy, and retinal remodeling (Marc et al., 2008). In this model 5-HT1A receptor agonists such as 8-hydroxy-DPATand buspirone were shown to 5-HT1A agonists provided potent and complete functional and structural protection. Protection was inhibited by treatment with WAY-100635, confirming the requirement for activating the 5-HT1A receptor in initiating this survival pathway. Consequently, agents acting as 5-HT1A agonist, including those addressed in this disclosure might be beneficial in the treatment of AMD.

### Cerebrovascular disorders

5-HT1A agonists have hypotensive effects so they may utility in the treatment of high blood pressure (Kubo et al., 1995). Furthermore these agents are likely to be useful for the prophylaxis and therapy of cerebral disorders (e.g. migraine), in particular in geriatrics, and for the control of the sequelae of cerebral infarcts (apoplexia cerebri), such as stroke and cerebral ischaemias.

### Endocrinology and gynaecology

Full or partial agonists of 5-HT1A receptors facilitate HPA excitability and, at higher doses, directly stimulate ACTH secretion (Korneyev, 1997). Furthermore, the use in endocrinology and gynecology is described, e.g. for the treatment of acromegaly, hypogonadism, secondary amenorrhea, premenstrual syndrome or undesired puerperal lactation. For example, prolactin responses to buspirone challenges were examined in seven women with late luteal phase dysphoric disorder (LLPDD) and the same number of healthy controls. The responses were found to be blunted during the follicular phase in the women with LLPDD (characterized by depressed or labile mood, anxiety, irritability, anger, and other symptoms occurring exclusively during the 2 weeks preceding menses) suggesting that 5-HT1A receptor subsensitivity might be an important trait in LLPDD sufferers (Yatham, 1993) that could be effectively treated with 5-HT1A agonist.

### Dermatology

Atopic dermatitis can be aggravated by mental stress and activation of 5-HT1A receptor subtype, has been shown to significantly inhibit stress-induced degranulation of mouse dermal mast cells (Kawana et al., 2010). Inhibition of stress-induced mast cell degranulation may be one of the mechanisms underlying the clinical efficacy. Therefore, 5-HT1A agonists are likely to be useful in the clinical management of stress-associated aggravation of atopic dermatitis.

### Gastroenterology

5-HT1A receptor agonists appear to be broad spectrum antiemetic agents (Wolff et al., 1997; Alfieri et al., 1995; Wolff et al., 1995; Lucot, 1997). Consequently the compounds reported here are likely to be useful in the treatment of emesis as well as prevention of disorders of the gastrointestinal tract associated with unnecessary or undesirable gastric acid secretion including peptic ulcer gastric and duodenal ulcers, gastritis, gastroesophogeal reflux disease, gastric dispepsia, and Zollinger-Ellison syndrome.

### References

Akimova E, Lanzenberger R, Kasper S (2009) The serotonin-1A receptor in anxiety disorders. Biological psychiatry 66:627-635.
Alfieri AB, Cubeddu LX (1995) Comparative efficacy of a single oral dose of ondansetron and of buspirone against cisplatin-induced emesis in cancer patients. British journal of cancer 72:1013-1015.
Anand A, Sharma K, Chen W, Sharma NK (2014). Using current data to define new approach in age related macular degeneration: need to accelerate translational research. Current genomics 15(4): 266-277.
Berrocoso E, De Benito MD, Mico JA (2007) Role of serotonin 5-HT1A and opioid receptors in the antiallodynic effect of tramadol in the chronic constriction injury model of neuropathic pain in rats. Psychopharmacology 193:97-105.
Blier P, El Mansari M (2013) Serotonin and beyond: therapeutics for major depression. Philosophical transactions of the Royal Society of London Series B, Biological sciences 368:20120536.
Caccia, S., 2007. N-dealkylation of arylpiperazine derivatives: disposition and metabolism of the 1-aryl-piperazines formed. Curr. Drug Metab. 8, 612-622.
Choi IS, Cho JH, Jang IS (2013) 5-Hydroxytryptamine 1A receptors inhibit glutamate release in rat medullary dorsal horn neurons. Neuroreport 24:399-403.
Citrome L (2012) Lurasidone for the acute treatment of adults with schizophrenia: what is the number needed to treat, number needed to harm, and likelihood to be helped or harmed? Clinical schizophrenia & related psychoses 6:76-85.
Coates MD, Mahoney CR, Linden DR, Sampson JE, Chen J, Blaszyk H, Crowell MD, Sharkey KA, Gershon MD, Mawe GM, Moses PL (2004) Molecular defects in mucosal serotonin content and decreased serotonin reuptake transporter in ulcerative colitis and irritable bowel syndrome. Gastroenterology 126:1657-1664.
Colpaert FC (2006) 5-HT(1A) receptor activation: new molecular and neuroadaptive mechanisms of pain relief. Curr Opin Investig Drugs 7:40-47.
Dourish CT, Kennett GA, Curzon G (1987) The 5-HT1A agonists 8-OH-DPAT, buspirone and ipsapirone attenuate stress-induced anorexia in rats. J Psychopharmacol 1:23-30.
Druse, M., Tajuddin, N.F., Gillespie, R.A., Le, P., 2005. Signaling pathways involved with serotonin1A agonist-mediated neuroprotection against ethanol-induced apoptosis of fetal rhombencephalic neurons. Brain Res. Dev. Brain Res. 159, 18-28.
Fuller RW (1991) Role of serotonin in therapy of depression and related disorders. The Journal of clinical psychiatry 52 Suppl: 52-57.
Fuss J, Vogt MA, Weber KJ, Burke TF, Gass P, Hensler JG (2013) Hippocampal serotonin-1A receptor function in a mouse model of anxiety induced by long-term voluntary wheel running. Synapse 67:648-655.
Gershon MD (2004) Review article: serotonin receptors and transporters -- roles in normal and abnormal gastrointestinal motility. Alimentary pharmacology & therapeutics 20 Suppl 7:3-14.
Gershon MD (2005) Nerves, reflexes, and the enteric nervous system: pathogenesis of the irritable bowel syndrome. Journal of clinical gastroenterology 39:S184-193.
Goetz CG, Damier P, Hicking C, Laska E, Muller T, Olanow CW, Rascol O, Russ H (2007) Sarizotan as a treatment for dyskinesias in Parkinson's disease: a double-blind placebo-controlled trial. Movement disorders: official journal of the Movement Disorder Society 22:179-186.
Hagan MM, Moss DE (1993) Effect of naloxone and antidepressants on hyperphagia produced by peptide YY. Pharmacology, biochemistry, and behavior 45:941-944.
Huot P, Fox SH, Newman-Tancredi A, Brotchie JM (2011) Anatomically selective serotonergic type 1A and serotonergic type 2A therapies for Parkinson's disease: an approach to reducing dyskinesia without exacerbating parkinsonism? The Journal of pharmacology and experimental therapeutics 339:2-8.
Idova GV, Davydova SM (2010) Involvement of presynaptic 5-HT(1A) receptors in immunomodulation in conditions of psychoemotional tension. Neuroscience and behavioral physiology 40:495-499.
Iravani MM, Jackson MJ, Kuoppamaki M, Smith LA, Jenner P (2003) 3,4-methylenedioxymethamphetamine (ecstasy) inhibits dyskinesia expression and normalizes motor activity in 1-methyl-4-phenyl-1,2,3,6-tetrahydropyridine-treated primates. The Journal of neuroscience: the official journal of the Society for Neuroscience 23:9107-9115.
Iravani MM, Tayarani-Binazir K, Chu WB, Jackson MJ, Jenner P (2006) In 1-methyl-4-phenyl-1,2,3,6-tetrahydropyridine-treated primates, the selective 5-hydroxytryptamine 1a agonist (R)-(+)-8-OHDPAT inhibits levodopa-induced dyskinesia but only with\ increased motor disability. The Journal of pharmacology and experimental therapeutics 319:1225-1234.
Jann MW, ZumBrunnen TL, Tenjarla SN, Ward ES, Jr., Weidler DJ (1998) Relative bioavailability of ondansetron 8-mg oral tablets versus two extemporaneous 16-mg suppositories: formulation and gender differences. Pharmacotherapy 18:288-294.
Jenike MA, Baer L, Buttolph L (1991) Buspirone augmentation of fluoxetine in patients with obsessive compulsive disorder. The Journal of clinical psychiatry 52:13-14.
Kanai A, Suzuki A, Osawa S, Hoka S (2006) Sumatriptan alleviates pain in patients with trigeminal neuralgia. The Clinical journal of pain 22:677-680.
Kannari K, Kurahashi K, Tomiyama M, Maeda T, Arai A, Baba M, Suda T, Matsunaga M (2002) [Tandospirone citrate, a selective 5-HT1A agonist, alleviates L-DOPA-induced dyskinesia in patients with Parkinson's disease]. No to shinkei = Brain and nerve 54:133-137.
Kawana S, Kato Y, Omi T (2010) Efficacy of a 5-HT1a receptor agonist in atopic dermatitis. Clinical and experimental dermatology 35:835-840.
King MV, Marsden CA, Fone KC (2008) A role for the 5-HT(1A), 5-HT4 and 5-HT6 receptors in learning and memory. Trends in pharmacological sciences 29:482-492.
Klempin F, Babu H, De Pietri Tonelli D, Alarcon E, Fabel K, Kempermann G (2010) Oppositional effects of serotonin receptors 5-HT1a, 2, and 2c in the regulation of adult hippocampal neurogenesis. Frontiers in molecular neuroscience 3.
Koprivica, V., Regardie, K., Wolff, C., Fernalld, R., Murphy, J.J., Kambayashi, J., Kikuchi, T., Jordan, S., 2011. Aripiprazole protects cortical neurons from glutamate toxicity. Eur. J. Pharmacol. 651, 73-76.
Korneyev AY (1997) The role of the hypothalamic-pituitary-adrenocortical axis in memory-related effects of anxiolytics. Neurobiology of learning and memory 67:1-13.
Koroshetz WJ, Moskowitz MA (1996) Emerging treatments for stroke in humans. Trends in pharmacological sciences 17:227-233.
Kubo T, Taguchi K, Ozaki S, Amano M, Ishizuka T (1995) 8-OH-DPAT-induced hypotensive action and sympathoexcitatory neurons in the rostral ventrolateral medulla of the rat. Brain research bulletin 36:405-411.
Lee YH, Choi SJ, Ji JD, Song GG (2012) Candidate gene studies of fibromyalgia: a systematic review and meta-analysis. Rheumatology international 32:417-426.
Lemonde S, Turecki G, Bakish D, Du L, Hrdina PD, Bown CD, Sequeira A, Kushwaha N, Morris SJ, Basak A, Ou XM, Albert PR (2003) Impaired repression at a 5-hydroxytryptamine 1A receptor gene polymorphism associated with major depression and suicide. The Journal of neuroscience: the official journal of the Society for Neuroscience 23:8788-8799.
Lesch KP (1991) 5-HT1A receptor responsivity in anxiety disorders and depression. Progress in neuro-psychopharmacology & biological psychiatry 15:723-733.
Lieb K, Fiebich BL, Herpfer I, Mantovani M, Loffler M, Feuerstein TJ (2005) No modulatory effect of neurokinin-1 receptor antagonists on serotonin uptake in human and rat brain synaptosomes. European neuropsychopharmacology: the journal of the European College of Neuropsychopharmacology 15:641-646.
Lou JS, Goldfarb L, McShane L, Gatev P, Hallett M (1995) Use of buspirone for treatment of cerebellar ataxia. An open-label study. Archives of neurology 52:982-988.
Lucki I (1998) The spectrum of behaviors influenced by serotonin. Biological psychiatry 44:151-162.
Lucot JB, Obach RS, McLean S, Watson JW (1997) The effect of CP-99994 on the responses to provocative motion in the cat. British journal of pharmacology 120:116-120.
Manto MU (2005) The wide spectrum of spinocerebellar ataxias (SCAs). Cerebellum 4:2-6.
Mantovani M, Bubl B, Feuerstein TJ (2006) 5-HT uptake blockade prevents the increasing effect of K(ATP) channel blockers on electrically evoked [3H]-5-HT release in rat and mouse neocortical slices. Neurochemistry international 48:218-225.
Marc RE, Jones BW, Watt CB, Vazquez-Chona F, Vaughan DK, Organisciak DT (2008). Extreme retinal remodeling triggered by light damage: implications for age related macular degeneration. Molecular vision 14: 782-806.
Matsuo, T., Izumi, Y., Kume, T., Takada-Takatori, Y., Sawada, H., Akaike, A., 2010. Protective effect of aripiprazole against glutamate cytotoxicity in dopaminergic neurons of rat mesencephalic cultures. Neurosci. Lett. 481, 78-81.
Meltzer HY, Maes M (1995) Pindolol pretreatment blocks stimulation by meta-chlorophenylpiperazine of prolactin but not cortisol secretion in normal men. Psychiatry research 58:89-98.
Moss LE, Neppe VM, Drevets WC (1993) Buspirone in the treatment of tardive dyskinesia. Journal of clinical psychopharmacology 13:204-209.
Mossner R, Lesch KP (1998) Role of serotonin in the immune system and in neuroimmune interactions. Brain, behavior, and immunity 12:249-271.
Murphy RM, Zemlan FP (1990) Selective serotonin1A/1B agonists differentially affect spinal nociceptive reflexes. Neuropharmacology 29:463-468.
Newman-Tancredi A, Kleven MS (2011) Comparative pharmacology of antipsychotics possessing combined dopamine D2 and serotonin 5-HT1A receptor properties. Psychopharmacology 216:451-473.
Nita M, Grzybowski A, Ascaso FJ, Huerva V (2014). Age-Related Macular Degeneration in the Aspect of Chronic Low-Grade Inflammation (Pathophysiological Paralnflammation). Mediators of inflammation 2014: 930671.
Nugent AC, Bain EE, Carlson PJ, Neumeister A, Bonne O, Carson RE, Eckelman W, Herscovitch P, Zarate CA, Jr., Charney DS, Drevets WC (2013) Reduced postsynaptic serotonin type 1A receptor binding in bipolar depression. European neuropsychopharmacology: the journal of the European College of Neuropsychopharmacology 23:822-829.
Nugent AC, Carlson PJ, Bain EE, Eckelman W, Herscovitch P, Manji H, Zarate CA, Jr., Drevets WC (2013) Mood stabilizer treatment increases serotonin type 1A receptor binding in bipolar depression. J Psychopharmacol 27:894-902.
Overstreet DH, Daws LC, Schiller GD, Orbach J, Janowsky DS (1998) Cholinergic/serotonergic interactions in hypothermia: implications for rat models of depression. Pharmacology, biochemistry, and behavior 59:777-785.
Papakostas GI, Petersen TJ, Kinrys G, Burns AM, Worthington JJ, Alpert JE, Fava M, Nierenberg AA (2005) Aripiprazole augmentation of selective serotonin reuptake inhibitors for treatment-resistant major depressive disorder. The Journal of clinical psychiatry 66:1326-1330.
Park, S.W., Lee, C.H., Lee, J.G., Kim, L.W., Shin, B.S., Lee, B.J., Kim, Y.H., 2011. Protective effects of atypical antipsychotic drugs against MPP(+)-induced oxidative stress in PC12 cells. Neurosci. Res. 69, 283-290.
Park, S.W., Lee, J.G., Ha, E.K., Choi, S.M., Cho, H.Y., Seo, M.K., Kim, Y.H., 2009. Differential effects of aripiprazole and haloperidol on BDNF-mediated signal changes in SH-SY5Y cells. Eur. Neuropsychopharmacol. 19, 356-362.
Pato MT, Pigott TA, Hill JL, Grover GN, Bernstein S, Murphy DL (1991) Controlled comparison of buspirone and clomipramine in obsessive-compulsive disorder. The American journal of psychiatry 148:127-129.
Pedotti R, Mitchell D, Wedemeyer J, Karpuj M, Chabas D, Hattab EM, Tsai M, Galli SJ, Steinman L (2001) An unexpected version of horror autotoxicus: anaphylactic shock to a self-peptide. Nature immunology 2:216-222.
Politis M (2010) Dyskinesias after neural transplantation in Parkinson's disease: what do we know and what is next? BMC medicine 8:80.
Politis M, Loane C (2011) Serotonergic dysfunction in Parkinson's disease and its relevance to disability. TheScientificWorldJournal 11:1726-1734.
Politis M, Oertel WH, Wu K, Quinn NP, Pogarell O, Brooks DJ, Bjorklund A, Lindvall O, Piccini P (2011) Graft-induced dyskinesias in Parkinson's disease: High striatal serotonin/dopamine transporter ratio. Movement disorders: official journal of the Movement Disorder Society 26:1997-2003.
Politis M, Wu K, Loane C, Quinn NP, Brooks DJ, Rehncrona S, Bjorklund A, Lindvall O, Piccini P (2010) Serotonergic neurons mediate dyskinesia side effects in Parkinson's patients with neural transplants. Science translational medicine 2:38ra46.
Politis M, Wu K, Loane C, Turkheimer FE, Molloy S, Brooks DJ, Piccini P (2010) Depressive symptoms in PD correlate with higher 5-HTT binding in raphe and limbic structures. Neurology 75:1920-1927.
Preece MA, Dalley JW, Theobald DE, Robbins TW, Reynolds GP (2004) Region specific changes in forebrain 5-hydroxytryptamine1A and 5-hydroxytryptamine2A receptors in isolation-reared rats: an in vitro autoradiography study. Neuroscience 123:725-732.
Rylander D (2012) The serotonin system: a potential target for anti-dyskinetic treatments and biomarker discovery. Parkinsonism & related disorders 18 Suppl 1:S126-128.
Song BM, Avery L (2012) Serotonin activates overall feeding by activating two separate neural pathways in Caenorhabditis elegans. The Journal of neuroscience: the official journal of the Society for Neuroscience 32:1920-1931.
Song W, Zukor H, Lin SH, Hascalovici J, Liberman A, Tavitian A, Mui J, Vali H, Tong XK, Bhardwaj SK, Srivastava LK, Hamel E, Schipper HM (2012) Schizophrenia-like features in transgenic mice overexpressing human HO-1 in the astrocytic compartment. The Journal of neuroscience: the official journal of the Society for Neuroscience 32:10841-10853.
Soumier A, Banasr M, Goff LK, Daszuta A (2010) Region- and phase-dependent effects of 5-HT(1A) and 5-HT(2C) receptor activation on adult neurogenesis. European neuropsychopharmacology: the journal of the European College of Neuropsychopharmacology 20:336-345.
Stanley MA, Turner SM, Borden JW (1990) Schizotypal features in obsessive-compulsive disorder. Comprehensive psychiatry 31:511-518.
Sukalović, V., Zlatović, M., Andrić, D., Roglić, G., Kostić-Rajacić, S., Soskić, V., 2005. Interaction of arylpiperazines with the dopamine receptor D2 binding site. Arzneimittelforschung. 55,145-152.
Suzuki M, Matsuda T, Asano S, Somboonthum P, Takuma K, Baba A (1995) Increase of noradrenaline release in the hypothalamus of freely moving rat by postsynaptic 5-hydroxytryptaminelA receptor activation. British journal of pharmacology 115:703-711.
Tovilovic, G., Zogovic, N., Harhaji-Trajkovic, L., Misirkic-Marjanovic, M., Janjetovic, K., Vucicevic, L., Kostic-Rajacic, S., Schrattenholz, A., Isakovic, A., Soskic, V., Trajkovic, V., 2012. Arylpiperazine dopamineric ligands protect neuroblastoma cells from nitric oxide (NO)-induced mitochondrial damage and apoptosis. ChemMedChem. 7, 495-508.
Tovilovic G, Zogovic N, Soskic V, Schrattenholz A, Kostic-Rajacic S, Misirkic-Marjanovic M, Janjetovic K, Vucicevic L, Arsikin K, Harhaji-Trajkovic L, Trajkovic V. 2013. Arylpiperazine-mediated activation of Akt protects SH-SY5Y neuroblastoma cells from 6-hydroxydopamine-induced apoptotic and autophagic death. Neuropharmacology. 72C, 224-235.
Trouillas P, Xie J, Getenet JC, Adeleine P, Nighoghossian N, Honnorat J, Riche G, Derex L (1995) [Effect of buspirone, a serotonergic 5-HT-1A agonist in cerebellar ataxia: a pilot study. Preliminary communication]. Revue neurologique 151:708-713.
Valhondo M, Marco I, Martin-Fontecha M, Vazquez-Villa H, Ramos JA, Berkels R, Lauterbach T, Benhamu B, Lopez-Rodri Guez ML (2013) New Serotonin 5-HT1A Receptor Agonists Endowed with Antinociceptive Activity in Vivo. Journal of medicinal chemistry.
Van Ameringen M, Mancini C, Wilson C (1996) Buspirone augmentation of selective serotonin reuptake inhibitors (SSRIs) in social phobia. Journal of affective disorders 39:115-121.
van Praag H, Black IB, Staubli UV (1997) Neonatal vs. adult unilateral hippocampal lesions: differential alterations in contralateral hippocampal theta rhythm. Brain research 768:233-241.
van Praag HM (1997) [Treatment of refractory primary depression]. Nederlands tijdschrift voor geneeskunde 141 :1375-1379.
Wilson SJ, Bailey JE, Rich AS, Nash J, Adrover M, Tournoux A, Nutt DJ (2005) The use of sleep measures to compare a new 5HT1A agonist with buspirone in humans. J Psychopharmacol 19:609-613.
Wolff MC, Leander JD (1997) Effects of a 5-HT1A receptor agonist on acute and delayed cyclophosphamide-induced vomiting. European journal of pharmacology 340:217-220.
Yatham LN (1993) Is 5HT1A receptor subsensitivity a trait marker for late luteal phase dysphoric disorder? A pilot study. Canadian journal of psychiatry Revue canadienne de psychiatrie 38:662-664.

## Claims

1. A compound of the general formula A 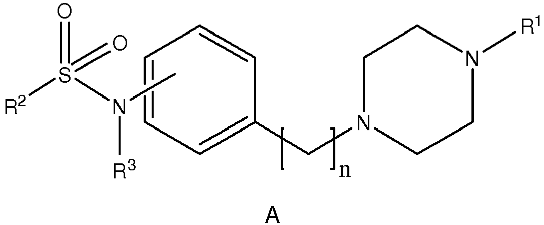 or a or a pharmaceutically acceptable salt or solvate thereof,
wherein:
R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³;
R² is selected from the group consisting of: C₆-C₁₀-aryl and C₅-C₇-heteroaryl; each optionally substituted with halogen, hydroxyl, OCOR³ amino, C₁-C₆-alkylamino, C₁-C₆-dialkyl-amino, C₁-C₆-(halo)alkyl, C₁-C₆-(halo)alkoxy and/or COOR³;
R³ is independently at each occurrence selected from the group consisting of: hydrogen and C₁-C₂-alkyl; and
n is an integer from 2 to 10 inclusive.

2. A compound as claimed in Claim 1 wherein R¹ is phenyl, which is optionally substituted with halogen, hydroxyl, and/or C₁-C₆-alkoxy; R² is selected from the group consisting of: phenyl, 2-pyridyl, 3-pyridyl, 4-pyridyl, pyridazinyl, pyrazinyl, each optionally substituted with halogen, hydroxyl, amino and/or C₁-C₆-alkoxy; and n is 2.

3. A compound as claimed in Claim 1 wherein R¹ is an unsubstituted phenyl; R² is selected from the group consisting of: phenyl, 2-pyridyl, 3-pyridyl, 4-pyridyl, pyridazinyl, pyrazinyl, each optionally substituted with hydroxyl; R³ is hydrogen; and n is 2.

4. A compound as claimed in any preceding claim wherein the general formula A comprises a compound of the general formula I.

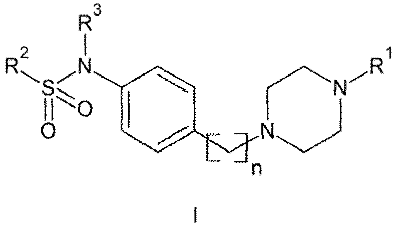

5. A compound as claimed in any preceding claim wherein the general formula A comprises a compound of the general formula II.

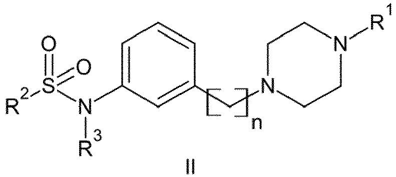

6. A compound as claimed in any preceding claim wherein the general formula A comprises a compound of the general formula III.

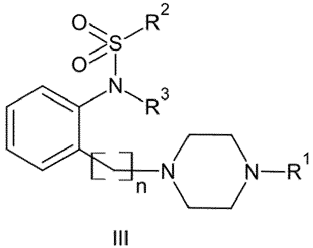

7. A compound as claimed in Claim 1 wherein the compound is selected from the group consisting of:
N-{4-[2-(4-phenyl-piperazin-1-yl)-ethyl]-phenyl}-benzsulphonamide,
N-{3-[2-(4-phenyl-piperazin-1-yl)-ethyl]-phenyl}-benzsulphonamide,
N-{2-[2-(4-phenyl-piperazin-1-yl)-ethyl]-phenyl}-benzsulphonamide,
N-{4-[2-(4-phenyl-piperazin-1-yl)-ethyl]-phenyl}-2-pyridylsulphonamide,
N-{3-[2-(4-phenyl-piperazin-1-yl)-ethyl]-phenyl}-2-pyridylsulphonamide,
N-{2-[2-(4-phenyl-piperazin-1-yl)-ethyl]-phenyl}-2-pyridylsulphonamide,
N-{4-[2-(4-phenyl-piperazin-1-yl)-ethyl]-phenyl}-3-pyridylsulphonamide,
N-{3-[2-(4-phenyl-piperazin-1-yl)-ethyl]-phenyl}-3-pyridylsulphonamide,
N-{2-[2-(4-phenyl-piperazin-1-yl)-ethyl]-phenyl}-3-pyridylsulphonamide,
and pharmaceutically acceptable salts or solvates thereof.

8. A compound as claimed in any preceding claim having neuroprotective activity.

9. A compound as claimed in any preceding claim being capable of protecting human neuroblastoma cells from oxidative stress induced by 6-hydroxy dopamine.

10. A method for the manufacture of a compound as claimed in any preceding claim, which comprises the steps of:
(i) reacting an arylcarboxylic acid of general formula IV a), IV b), or IV c) 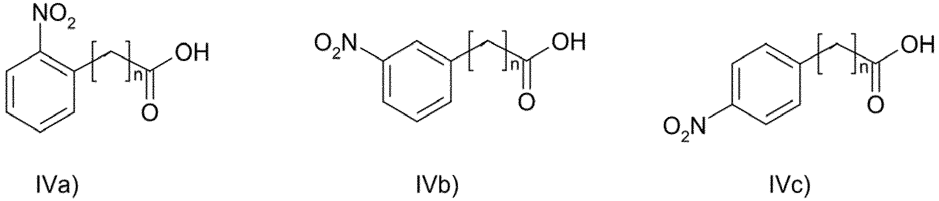 with an amine of general formula V 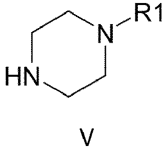 to give a compound of general formula VIa), VIb), or VIc); 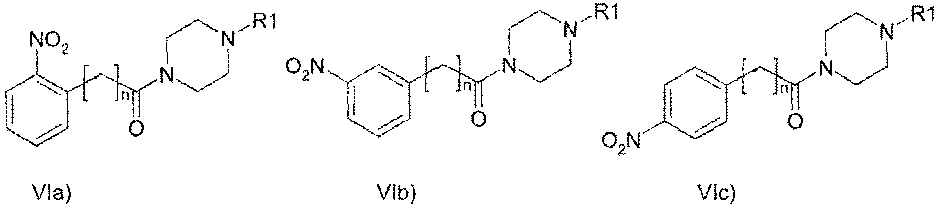
(ii) reducing said compound of formula VI a), VI b) or VI c) with a suitable reducing agent;
(iii) hydrogenating the product of step (ii);
(iv) reacting the product of step (iii) with a arylsulphonic acid chloride of the general formula R²SO₂Cl; and
(v) optionally isolating the resulting compound of formula A, I, II, or III,
wherein:
R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³;
R² is selected from the group consisting of: C₆-C₁₀-aryl and C₅-C₇-heteroaryl; each optionally substituted with halogen, hydroxyl, OCOR³ amino, C₁-C₆-alkylamino, C₁-C₆-dialkyl- amino, C₁-C₆-(halo)alkyl, C₁-C₆-(halo)alkoxy and/or COOR³;
R³ is independently at each occurrence selected from the group consisting of: hydrogen and C₁-C₂-alkyl; and
n is an integer from 1 to 9 inclusive.

11. A method as claimed in Claim 10 wherein the arylcarboxylic acid of step (i) has the general formula VII 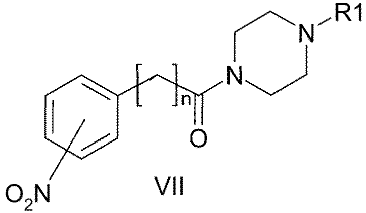
wherein R¹ is phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³; and n is an integer from 1 to 9 inclusive; and
wherein the amine of step (i) has the general formula VIII 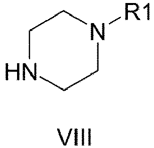 wherein R¹ phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³.

12. A method as claimed in Claim 10 or Claim 11 wherein the reaction product of step (i) has the general formula IX 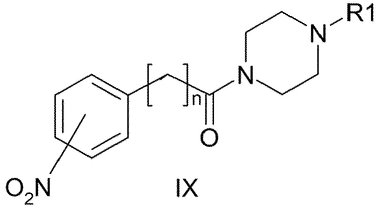 wherein R¹ phenyl optionally substituted with halogen, hydroxyl, OCOR³, amino, C₁-C₆-alkylamino, C₁-C₆-dialkylamino, C₁-C₆-(halo)alkyl, C₁-C₆-(halo)- alkoxy and/or COOR³.

13. A method as claimed in Claim 12 wherein R¹ is phenyl, R² is selected from phenyl, 2-pyridyl, 3-pyridyl, 4-pyridyl, pyridazinyl, and pyrazinyl, each optionally substituted with hydroxyl; R³ is hydrogen at each occurrence; and n is 1.

14. A pharmaceutical composition comprising one or more compounds as claimed in any of claims 1 to 9 and pharmaceutically acceptable excipients, adjuvants, diluents and/or carriers.

15. A compound as claimed in any of claims 1 to 9 or a pharmaceutical composition as claimed in Claim 14 for use as a medicament.

## Patentansprüche

1. Verbindung der allgemeinen Formel A 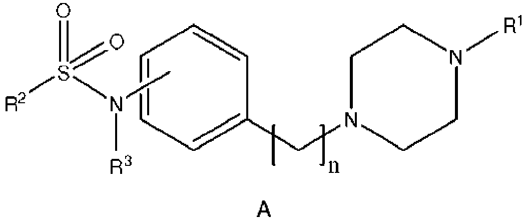 oder ein pharmazeutisch annehmbares Salz oder Solvat davon, wobei:
R¹ Phenyl ist, das optional mit Halogen, Hydroxyl, OCOR³, Amino, C₁-C₆-Alkylamino, C₁-C₆-Dialkylamino, C₁-C₆-(Halo)alkyl, C₁-C₆-(Halo)alkoxy und/oder COOR³ substituiert ist;
R² aus der Gruppe ausgewählt ist, die aus Folgendem besteht: C₆-C₁₀-Aryl und C₅-C₇-Heteroaryl; jeweils optional mit Halogen, Hydroxyl, OCOR³-Amino, C₁-C₆-Alkylamino, C₁-C₆-Dialkylamino, C₁-C₆-(Halo)alkyl, C₁-C₆-(Halo)alkoxy und/oder COOR³ substituiert;
R³ unabhängig bei jedem Auftreten aus der Gruppe ausgewählt ist, die aus Folgendem besteht: Wasserstoff und C₁-C₂-Alkyl; und
n eine ganze Zahl von 2 bis einschließlich 10 ist.

2. Verbindung nach Anspruch 1, wobei R¹ Phenyl ist, das optional mit Halogen, Hydroxyl und/oder C₁-C₆-Alkoxy substituiert ist; R² aus der Gruppe ausgewählt ist, die aus Folgendem besteht: Phenyl, 2-Pyridyl, 3-Pyridyl, 4-Pyridyl, Pyridazinyl, Pyrazinyl, jeweils optional mit Halogen, Hydroxyl, Amino und/oder C₁-C₆-Alkoxy substituiert; und wobei n 2 ist.

3. Verbindung nach Anspruch 1, wobei R¹ ein nichtsubstituiertes Phenyl ist; R² aus der Gruppe ausgewählt ist, die aus Folgendem besteht: Phenyl, 2-Pyridyl, 3-Pyridyl, 4-Pyridyl, Pyridazinyl, Pyrazinyl, jeweils optional mit Hydroxyl substituiert; wobei R³ Wasserstoff ist; und n 2 ist.

4. Verbindung nach einem der vorstehenden Ansprüche, wobei die allgemeine Formel A eine Verbindung der allgemeinen Formel I umfasst.

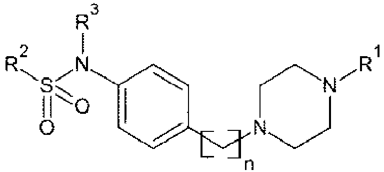

5. Verbindung nach einem der vorstehenden Ansprüche, wobei die allgemeine Formel A eine Verbindung der allgemeinen Formel II umfasst.

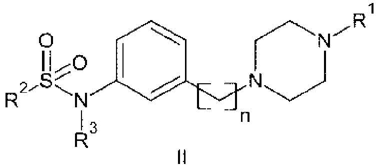

6. Verbindung nach einem der vorstehenden Ansprüche, wobei die allgemeine Formel A eine Verbindung der allgemeinen Formel III umfasst.

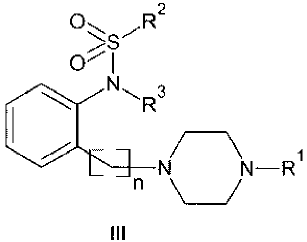

7. Verbindung nach Anspruch 1, wobei die Verbindung aus der Gruppe ausgewählt ist, die aus Folgendem besteht:
N-{4-[2-(4-Phenylpiperazin-1-yl)ethyl]phenyl}benzsulphonamid,
N-{3-[2-(4-Phenylpiperazin-1-yl)ethyl]phenyl}benzsulphonamid,
N-{2-[2-(4-Phenylpiperazin-1-yl)ethyl]phenyl}benzsulphonamid,
N-{4-[2-(4-Phenylpiperazin-1-yl)ethyl]phenyl}-2-pyridylsulphonamid,
N-{3-[2-(4-Phenylpiperazin-1-yl)ethyl]phenyl}-2-pyridylsulphonamid,
N-{2-[2-(4-Phenylpiperazin-1-yl)ethyl]phenyl}-2-pyridylsulphonamid,
N-{4-[2-(4-Phenylpiperazin-1-yl)ethyl]phenyl}-3-pyridylsulphonamid,
N-{3-[2-(4-Phenylpiperazin-1-yl)ethyl]phenyl}-3-pyridylsulphonamid,
N-{2-[2-(4-Phenylpiperazin-1-yl)ethyl]phenyl}-3-pyridylsulphonamid,
und pharmazeutisch annehmbaren Salzen oder Solvaten davon.

8. Verbindung nach einem der vorstehenden Ansprüche, die eine neuroprotektive Wirksamkeit aufweist.

9. Verbindung nach einem der vorstehenden Ansprüche, die in der Lage ist, menschliche Neuroblastomzellen vor oxidativem Stress, der durch 6-Hydroxydopamin induziert wird, zu schützen.

10. Verfahren zum Herstellen einer Verbindung nach einem der vorstehenden Ansprüche, das die folgenden Schritte umfasst:
(i) Reagierenlassen einer Arylcarboxylsäure der allgemeinen Formel IVa), IVb) oder IVc) 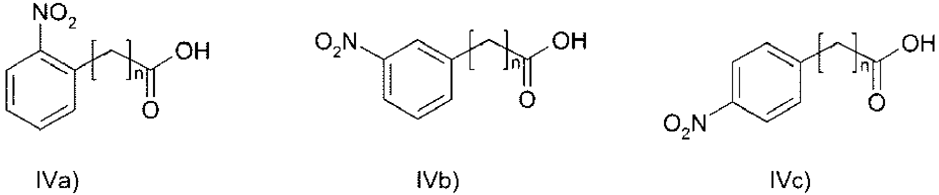 mit einem Amin der allgemeinen Formel V, 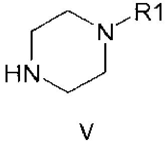 um eine Verbindung der allgemeinen Formel VIa), VIb) oder VIc) zu erhalten; 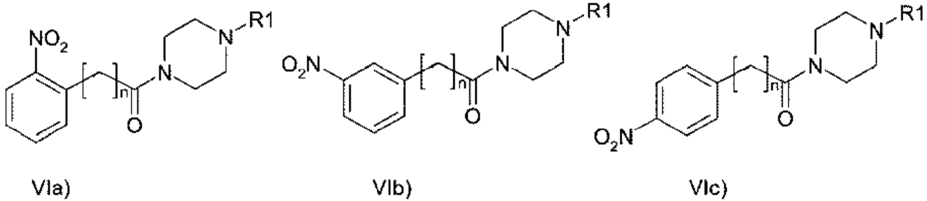
(ii) Reduzieren der Verbindung der Formel VI a), VI b) oder VI c) mit einem geeigneten Reduktionsmittel;
(iii) Hydrieren des Produktes aus Schritt (ii);
(iv) Reagierenlassen des Produktes aus Schritt (iii) mit einem Arylsulfonsäurechlorid der allgemeinen Formel R²SO₂Cl; und
(v) optional Isolieren der resultierenden Verbindung der Formel A, I, II oder III, wobei:
R¹ Phenyl ist, das optional mit Halogen, Hydroxyl, OCOR³, Amino, C₁-C₆-Alkylamino, C₁-C₆-Dialkylamino, C₁-C₆-(Halo)alkyl, C₁-C₆-(Halo)alkoxy und/oder COOR³ substituiert ist;
R² ausgewählt ist aus der Gruppe bestehend aus: C₆₋C₁₀-Aryl und C₅-C₇-Heteroaryl, jeweils optional mit Halogen, Hydroxyl, OCOR³-Amino, C₁-C₆-Alkylamino, C₁-C₆-Dialkylamino, C₁-C₆-(Halo)alkyl, C₁-C₆-(Halo)alkoxy und/oder COOR³ substituiert;
R³ unabhängig bei jedem Auftreten aus der Gruppe ausgewählt ist, die aus Folgendem besteht: Wasserstoff und C₁-C₂-Alkyl; und
n eine ganze Zahl von 1 bis einschließlich 9 ist.

11. Verfahren nach Anspruch 10, wobei die Arylcarboxylsäure aus Schritt (i) die allgemeine Formel VII aufweist, 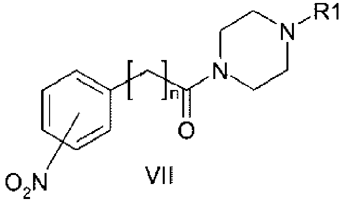
wobei R¹ Phenyl ist, das optional mit Halogen, Hydroxyl, OCOR³, Amino, C₁-C₆-Alkylamino, C₁-C₆-Dialkylamino, C₁-C₆-(Halo)alkyl, C₁-C₆-(Halo)alkoxy und/oder COOR³ substituiert ist; und n eine ganze Zahl von 1 bis einschließlich 9 ist; und
wobei das Amin aus Schritt (i) die allgemeine Formel VIII aufweist 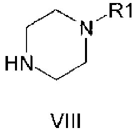 wobei R¹ Phenyl ist, das optional mit Halogen, Hydroxyl, OCOR³, Amino, C₁-C₆-Alkylamino, C₁-C₆-Dialkylamino, C₁-C₆-(Halo)alkyl, C₁-C₆-(Halo)alkoxy und/oder COOR³ substituiert ist.

12. Verfahren nach Anspruch 10 oder Anspruch 11, wobei das Reaktionsprodukt aus Schritt (i) die allgemeine Formel IX aufweist 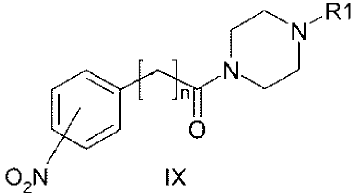 wobei R¹ Phenyl ist, das optional mit Halogen, Hydroxyl, OCOR³, Amino, C₁-C₆-Alkylamino, C₁-C₆-Dialkylamino, C₁-C₆-(Halo)alkyl, C₁-C₆-(Halo)alkoxy und/oder COOR³ substituiert ist.

13. Verfahren nach Anspruch 12, wobei R¹ Phenyl ist, R² ausgewählt ist aus Phenyl, 2-Pyridyl, 3-Pyridyl, 4-Pyridyl, Pyridazinyl und Pyrazinyl, jeweils optional substituiert mit Hydroxyl; wobei R³ an jedem Auftreten Wasserstoff ist; und n 1 ist.

14. Pharmazeutische Zusammensetzung, umfassend eine oder mehrere Verbindungen nach einem der Ansprüche 1 bis 9 und pharmazeutisch annehmbare Hilfsstoffe, Adjuvantien, Verdünnungsmittel und/oder Trägerstoffe.

15. Verbindung nach einem der Ansprüche 1 bis 9 oder eine pharmazeutische Zusammensetzung nach Anspruch 14 zur Verwendung als Medikament.

## Revendications

1. Composé de formule générale A 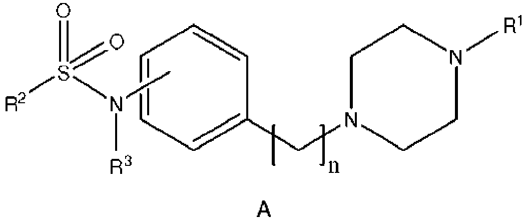 ou un sel ou un solvate pharmaceutiquement acceptable de celui-ci, dans lequel :
R¹ représente un groupe phényle éventuellement substitué par un halogène, un groupe hydroxyle, OCOR³, amino, C₁-C₆-alkylamino, C₁-C₆-dialkylamino, C₁-C₆-(halogéno)alkyle, C₁-C₆-(halogéno)-alcoxy et/ou COOR³ ;
R² est choisi dans le groupe constitué de : C₆-C₁₀-aryle et C₅-C₇-hétéroaryle ; chacun étant éventuellement substitué par un halogène, un groupe hydroxyle, OCOR³ amino, C₁-C₆-alkylamino, C₁-C₆-dialkyl-amino, C₁-C₆-(halogéno)alkyle,C₁-C₆-(halogéno)alcoxy et/ou COOR³ ;
R³ est indépendamment, à chaque occurrence, choisi dans le groupe constitué de : un atome d'hydrogène et un groupe C₁-C₂-alkyle ; et
n est un nombre entier valant entre 2 et 10 inclusivement.

2. Composé selon la revendication 1 dans lequel R¹ représente un groupe phényle, qui est éventuellement substitué par un halogène, un groupe hydroxyle, et/ou C₁-C₆-alcoxy ; R² est choisi dans le groupe constitué de : phényle, 2-pyridyle, 3-pyridyle, 4-pyridyle, pyridazinyle, pyrazinyle, chacun étant éventuellement substitué par un halogène, un groupe hydroxyle, amino et/ou C₁-C₆-alcoxy ; et n vaut 2.

3. Composé selon la revendication 1 dans lequel R¹ représente un groupe phényle non substitué ; R² est choisi dans le groupe constitué de : phényle, 2-pyridyle, 3-pyridyle, 4-pyridyle, pyridazinyle, pyrazinyle, chacun étant éventuellement substitué hydroxyle ; R³ représente un atome d'hydrogène ; et n vaut 2.

4. Composé selon l'une quelconque des revendications précédentes dans lequel la formule générale A comprend un composé qui répond à la formule générale I.

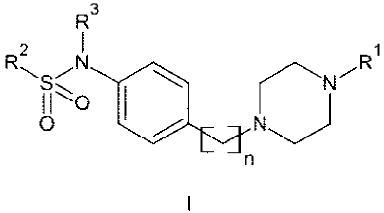

5. Composé selon l'une quelconque des revendications précédentes dans lequel la formule générale A comprend un composé qui répond à la formule générale II.

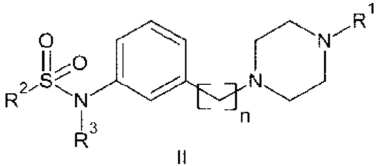

6. Composé selon l'une quelconque des revendications précédentes dans lequel la formule générale A comprend un composé qui répond à la formule générale III.

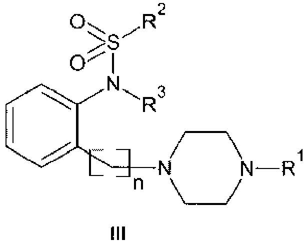

7. Composé selon la revendication 1 dans lequel le composé est choisi dans le groupe constitué de :
N-{4-[2-(4-phényl-pipérazin-1-yl)-éthyl]-phényl}-benzsulphonamide,
N-{3-[2-(4-phényl-pipérazin-1-yl)-éthyl]-phényl}-benzsulphonamide,
N-{2-[2-(4-phényl-pipérazin-1-yl)-éthyl]-phényl}-benzsulphonamide,
N-{4-[2-(4-phényl-pipérazin-1-yl)-éthyl]-phényl}-2-pyridylsulphonamide,
N-{3-[2-(4-phényl-pipérazin-1-yl)-éthyl]-phényl}-2-pyridylsulphonamide,
N-{2-[2-(4-phényl-pipérazin-1-yl)-éthyl]-phényl}-2-pyridylsulphonamide,
N-{4-[2-(4-phényl-pipérazin-1-yl)-éthyl]-phényl}-3-pyridylsulphonamide,
N-{3-[2-(4-phényl-pipérazin-1-yl)-éthyl]-phényl}-3-pyridylsulphonamide,
N-{2-[2-(4-phényl-pipérazin-1-yl)-éthyl]-phényl}-3-pyridylsulphonamide,
et des sels ou des solvates pharmaceutiquement acceptables de ceux-ci.

8. Composé selon l'une quelconque des revendications précédentes comportant une activité neuroprotectrice.

9. Composé selon l'une quelconque des revendications précédentes étend capable de protéger les cellules humaines de neuroblastome du stress oxydatif induit par la 6-hydroxy dopamine.

10. Procédé de fabrication du composé selon l'une quelconque des revendications précédentes, qui comprend les étapes de :
(i) réaction d'un acide arylcarboxylique de formule générale IV a), IV b), ou IV c) 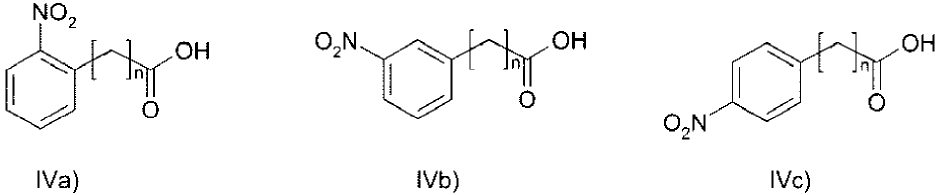 avec une amine de formule générale V 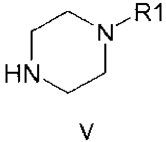 afin de donner un composé de formule générale VIa), VIb), ou VIc) ; 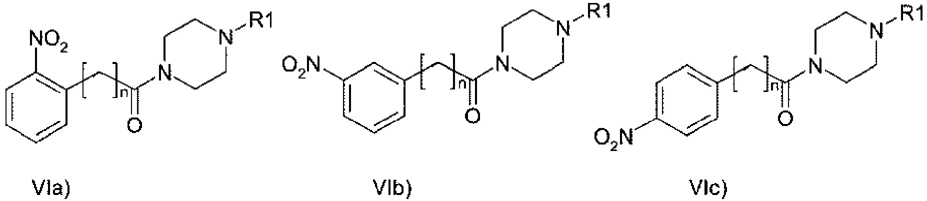
(ii) réduction dudit composée de formule VI a), VI b) ou VI c) avec un agent de réduction approprié ;
(iii) hydrogénation du produit de l'étape (ii) ;
(iv) réaction du produit de l'étape (iii) avec un chlorure d'acide arylsulphonique qui répond à la formule générale R²SO₂Cl ; et
(v) isolation éventuelle du composé résultant de formule A, I, II, ou III, dans lequel :
R¹ représente un groupe phényle éventuellement substitué par un halogène, un groupe hydroxyle, OCOR³, amino, C₁-C₆-alkylamino, C₁-C₆-dialkylamino, C₁-C₆-(halogéno)alkyle, C₁-C₆-(halogéno)-alcoxy et/ou COOR³ ;
R² est choisi dans le groupe constitué de : C₆-C₁₀-aryle et C₅-C₇-hétéroaryle ; chacun étant éventuellement substitué par un halogène, un groupe hydroxyle, OCOR³ amino, C₁-C₆-alkylamino, C₁-C₆-dialkyl-amino, C₁-C₆-(halogéno)alkyle,C₁-C₆-(halogéno)alcoxy et/ou COOR³ ;
R³ est indépendamment, à chaque occurrence, choisi dans le groupe constitué de : un atome d'hydrogène et un groupe C₁-C₂-alkyle ; et
n est un nombre entier valant entre 1 à 9 inclusivement.

11. Procédé selon la revendication 10 dans lequel l'acide arylcarboxylique de l'étape (i) répond à la formule générale VII 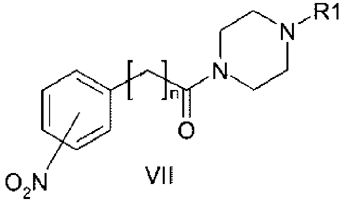
dans laquelle R¹ représente un groupe phényle éventuellement substitué par un halogène, un groupe hydroxyle, OCOR3, amino, C₁-C₆-alkylamino, C₁-C₆-dialkylamino, C₁-C₆-(halogéno)alkyle,C₁-C₆-(halogéno)-alcoxy et/ou COOR³ ; et n est un nombre entier valant entre 1 à 9 inclusivement ; et
dans lequel l'aminé de l'étape (i) répond à la formule générale VIII 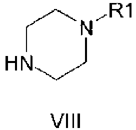 dans laquelle R¹ représente un groupe phényle éventuellement substitué par un halogène, un groupe hydroxyle, OCOR3, amino, C₁-C₆-alkylamino, C₁-C₆-dialkylamino, C₁-C₆-(halogéno)alkyle,C₁-C₆-(halogéno)-alcoxy et/ou COOR³.

12. Procédé selon la revendication 10 ou la revendication 11 dans lequel le produit de réaction de l'étape (i) répond à la formule générale IX 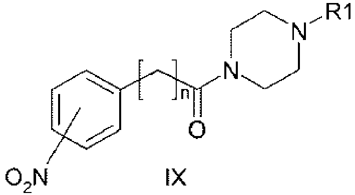 dans laquelle R¹ représente un groupe phényle éventuellement substitué par un halogène, un groupe hydroxyle, OCOR3, amino, C₁-C₆-alkylamino, C₁-C₆-dialkylamino, C₁-C₆-(halogéno)alkyle,C₁-C₆-(halogéno)-alcoxy et/ou COOR³.

13. Procédé selon la revendication 12 dans lequel R¹ représente un groupe phényle, R² est choisi parmi un groupe phényle, 2-pyridyle, 3-pyridyle, 4-pyridyle, pyridazinyle, et pyrazinyle, chacun étant éventuellement substitué par un groupe hydroxyle ; R³ représente un atome d'hydrogène à chaque occurrence ; et n vaut 1.

14. Composition pharmaceutique comprenant au moins un composé selon l'une quelconque des revendications 1 à 9 et des excipients, des adjuvants, des diluants et/ou des véhicules pharmaceutiquement acceptables.

15. Composé selon l'une quelconque des revendications 1 à 9 ou composition pharmaceutique selon la revendication 14 destiné(e) à être utilisé(e) en tant que médicament.
